# EUROPEAN PATENT APPLICATION

(11) **EP 4 702 988 A1**
(43) Date of publication of application: **04.03.2026**
(21) Application number: 24382927.2
(22) Date of filing: 28.08.2024
(51) Int. Cl.: A61K 39/008, A61P 33/02

(54) **COMBINATION PRODUCT FOR THE TREATMENT OF LEISHMANIASIS**

(71) Applicant: Biotechnology Assets S.A., 11591 Jerez de la Frontera Cádiz (ES); Consejo Superior de Investigaciones Científicas (CSIC), 41013 Sevilla (ES)
(72) Inventor: THOMAS CARAZO, María del Carmen, 18016 Granada (ES); LÓPEZ LÓPEZ, Manuel Carlos, 18016 Granada (ES); INFANTE VIÑOLO, Juan José, 11591 Jerez de la Frontera (ES); INFANTE VIÑOLO, Víctor Manuel, 11591 Jerez de la Frontera (ES); CID BOZA, Raquel, 11591 Jerez de la Frontera (ES)
(74) Representative: Hoffmann Eitle

(57) **Abstract**

The present application provides a combination product for the treatment and/or prevention of infections caused by parasites of the genus *Leishmania.*

## Description

### Technical field

The present application relates to a novel pharmaceutical combination product. The combination product and its components may be used as a medicament, in particular, as a medicament for the treatment and/or prevention of infections caused by parasites of the genus *Leishmania.*

### Background art

Species of the genus *Leishmania,* Protozoan intracellular parasites belonging to the order Kinetoplastida Trypanosomatidae family, are found in the tropical and subtropical regions of the world. These parasites cause a broad spectrum of clinical symptoms called leishmaniasis. Depending on the causative species, leishmaniasis can be cutaneous, mucocutaneous and/or visceral (in reference to their tissue tropism and the clinical symptoms). The incidence of leishmaniasis has increased enormously in recent years. Leishmaniasis is now endemic in 88 countries and it is claimed that there are 350 million people at risk of contracting the disease (WHO report, http://www.who.int/health-topics/leishmaniasis). It is estimated that there are about 12 million people affected by leishmaniasis worldwide. Of the 2 million cases of infections reported annually, 500,000 are visceral leishmaniasis (VL). This disease is caused by *L*. *infantum* and *L. donovani* and symptoms include intermittent fever, anemia, splenomegaly, hepatomegaly, and lymphadenopathy. The outcome of VL is usually death caused by a concomitant infection. Leishmania/HIV co-infection has been presented as a major health concern in recent years in Mediterranean countries. In these countries the main hosts are dogs, i.e. *Canis lupus familiaris,* which function as a natural reservoir of the parasite (Alvar et al. 2004 Adv Parasitol. 57: 1-88).

Vaccination is the most efficient medical treatment for the prevention of mortality and morbidity caused by infectious agents. Despite this, currently there are very few pathogenic microorganisms for which we have effective vaccines. In the case of species of the genus *Leishmania,* vaccines have been developed consisting of purified or recombinant fragments of *Leishmania*-derived antigens with variable success in recent years (Kedzierski et al. 2010. J Glob Infect Dis. 2 (2): 177-85; Kedzierski et al. 2006. Parasitology, 133: 87-112; Requena et al. 2004. Expert Opin Biol Ther. 4 (9): 1505-17). However, none of the tested vaccines have produced satisfactory results in humans so far.

Another option for treating leishmaniasis is immunotherapy or, in its absence, immunochemotherapy. Pasteurized attenuated parasites have been tested with adjuvant (Convit et al. 2003 Med Hyg, 97: 469-72), as well as a mixture of antigens which stem from the pathogen (Badaro et al. 2006 J Infect Dis, 194: 1151-9). However, success has been limited (El-On J. 2009. Isr Med Assoc J, 11 (10): 623-8).

As discussed earlier, dogs are natural reservoirs for some parasites of the genus *Leishmania.* Therefore, there have been several attempts to tackle this disease in dogs. In fact, leishmaniasis is a serious parasitic disease in dogs. The most common clinical symptom is hair-loss, especially around the eyes, ears, and nose. In a more advanced stage of the disease, the dog loses weight but not appetite and the dog has skin lesions, especially on the head and legs, as well as symptoms related to kidney failure. The disease is endemic in large parts of Spain, as well as most countries of the Mediterranean.

As in humans, there have been numerous attempts to treat and/or prevent the disease in dogs with very limited success (Alvar et al. 2004. Adv Parasitol. 57: 1-88; Reis et al. 2010 Trends Parasitol. 26 (7): 341-9).

For both human and canine leishmaniasis, one of the most promising treatments appears to be the use of DNA vaccines which encode for multiple *Leishmania*-specific antigens or the use of chimeric recombinant proteins containing different antigens of the parasite (De Oliveira et al. 2009 Parasitol Int. 58 (4): 319-24; Palatnik-de-Sousa, 2008. Vaccine 26: 1709-1724; WO 2013/011184).

However, when considering multiple experimental trials in dogs conducted previously by various authors, although vaccination with recombinant proteins encoding *Leishmania*-specific antigens partially protected dogs from exhibiting clinical symptoms of leishmaniasis, the vaccines generally failed to promote significant parasite clearance, as detected by PCR in the bone marrow of a majority of the dogs, specifically in 80% or more of the vaccinated dogs. The capacity for parasite clearance has been correlated with the ability of the vaccines to induce a T-cell specific cytotoxic response (Seyed et al. 2016, Front. Microbiol. 7:467; Thakur et al. 2022, Front. Immunol. 12:765684; Hurtado-Morillas et al. 2024, Animals, 14:605).

Regarding the limited capacity for parasite clearance induced by recombinant vaccine candidates, some researchers explored combining two proteins in the same vaccine formulation to improve the effectiveness of vaccination in experimentally infected dogs, compared to using a single recombinant protein (Moreno et al. 2007, Vaccine 25: 5290-5300). However, the results indicated a decrease in the effectiveness of the combination product compared to the single protein vaccines (Moreno et al. 2007, Vaccine 25: 5290-5300). A recombinant vaccine containing Leishmania recombinant protein histone 1 (H1) led to parasite clearance (PCR-negative) in the bone marrow in 75% of the vaccinated and infected dogs 48 weeks post-challenge. In contrast, a vaccine containing Leishmania recombinant protein hydrophilic acylated surface protein B1 (HASPB1) achieved parasite clearance in only 25% of the dogs. When the recombinant proteins H1 and HASPB1 were combined in the vaccine formulation, clearance rates were only 25% (Moreno et al. 2007, Vaccine 25: 5290-5300). The combination vaccine also performed worse than the single protein vaccines in preventing clinical symptoms 64 weeks post-challenge (Moreno et al. 2007, Vaccine 25: 5290-5300) (Table 1 and 2).

In another prior trial of a combination product as a prophylactic vaccine for dogs, vaccination with cathepsin L-like cysteine proteases CPA and CPB of Leishmania, combined with canine interleukin IL-12, did not protect dogs from experimental infection (Poot et al. 2006, Vaccine 24: 2460-2468) (Table 1 and 2).

PFR proteins (Paraflagellar Rod proteins) are a family of relevant specific antigens of trypanosomatids located in the paraflagellar region of these parasites (Fouts et al., 1998, J Biol Chem, 273(34): 21846-21855; Clark et al., 2005, Parasitol Res, 96(5): 312-320). Some members of this antigen family, specifically PFR1-3 proteins, are highly immunogenic (Michailowsky et al., Infect Immun, 71(6): 3165-3171). Immunization of mice with PFR1 and PFR2 proteins purified from T. cruzi induced a Th1 immune response capable of reducing the T. cruzi load in the heart tissue of immunized and infected mice (Morell et al., 2006, Vaccine, 24: 7046-7055). These results suggest that PFR proteins are suitable vaccine candidates. Previous research has shown that CD4+ lymphocytes isolated from mice immunized with PFR can activate parasite-infected macrophages, leading to parasite death through NO release (Wrightsman et al., 2000, Vaccine, 18(14): 1419-27; Miller et al., 1997, J Immunol, 158(11): 5330-7), and are also capable of reducing blood parasite levels in the absence of B cells, but not in the absence of functional CD4+ and CD8+ T lymphocytes. In terms of DNA vaccines, paraflagellar proteins of various kinetoplastids have been studied. The immunogenic and protective capacity of the L. mexicana PFR2 protein, when inoculated as a DNA vaccine and recombinant protein, has been demonstrated (Saravia et al., 2005, Vaccine, 23: 984-995). Immunization of mice with DNA vectors containing the gene encoding the PFR2 protein of T. cruzi, alone or fused to the Hsp70 protein of the same pathogen, induced high levels of IgG2a type anti-PFR antibodies. However, only immunization with the chimeric vaccine stimulated the production of IL-12 and IFN-γ, and led to a decrease in IL-4 producing cells, thereby triggering a protective response against T. cruzi (Morell et al., 2006, Vaccine, 24: 7046-7055).

Hsp70 belongs to the heat shock proteins (HSP) family, which are highly conserved across different species, both eukaryotic and prokaryotic. These proteins are essential for maintaining cellular homeostasis through their role as chaperones (Smith, Whitesell et al., 1998, Pharmacological Reviews, 50(4): 493-513). Furthermore, they are capable of inducing an immune response. Hsp70 proteins, when obtained from tumor cells or virus-infected cells, can activate the CD8+ CTL response both in vivo and in vitro against various antigens from the cells from which the Hsp70 proteins were purified (Srivastava, 2002, Nat Rev Immunol, 2: 185-194; Wu et al., 2005, Cancer Res., 65(11): 4947-4954). This capability is due to the fact that extracellular Hsp70 can form complexes with antigenic peptides and simultaneously activate antigen-presenting cells (APCs). This interaction triggers an immunological cascade that includes: the presentation of gathered peptides to CD8+ cells through MHC I and to CD4+ T cells via MHC II, the secretion of pro-inflammatory cytokines, and the functional and phenotypic maturation of dendritic cells (DCs) (Asea et al., 2000, Nat Med, 6: 435-442; Basu et al., 2001, Immunity, 14: 303-313; Harmala et al., 2002, J Immunol, 169: 5622-5629; Tobian et al., 2004, J Immunol, 173: 5130-5137).

*In vitro* tests conducted in our laboratory demonstrated that the Trypanosoma cruzi Hsp70 protein has a unique stimulatory effect on spleen and ganglion cells of naive mice, resulting in rapid and intense T-cell stimulation (Marañón et al., 2000, Int. Immunol., 12(12): 1685-1693). Additionally, this protein can induce a mixed immune response (IgG1 and IgG2a) against the associated hapten, which is independent of TLR2 and TLR4 receptors (Qazi et al., 2007, Vaccine, 25(6): 1096-1103). Hsp70 also triggered a specific response against KMP11 in mice when immunized with a vector encoding both proteins, activating the production of IgG2a-type specific antibodies against KMP 11 (Thomas, Olivares et al., 2000, DNA and Cell Biology, 19(1): 47-57; Thomas, Olivares et al., 2000, Acta Tropica, 75(2): 203-210). Moreover, mice immunized with the KMP1 1-Hsp70 fusion protein, but not those immunized with the isolated KMP11 protein, showed a cytotoxic lymphocyte response against Jurkat-A2/Kb cells expressing the KMP11 protein, as well as against cells loaded with KMP11-derived peptides with affinity for the A2 molecule. Similar results have been achieved by administering Hsp70 together with the gp63 metalloproteinase of Leishmania donovani (Kaur et al., 2011, Parasite Immunol., 33(2): 95-103).

Parasite clearance is a crucial characteristic desired in the immune response induced by any vaccine candidate against leishmaniasis, going beyond merely preventing the development of clinical signs. Dogs serve as reservoirs for the parasite. Infected dogs, even if asymptomatic, can transmit the parasite to humans via the Phlebotominae vector. Therefore, in addition to preventing the onset of symptoms and disease progression, an effective vaccine candidate should also promote a significant reduction in the parasite load after infection in dogs or humans. This reduction should be sufficient to lower the risk of transmission from the infected host to the Phlebotominae vector (Molina et al., 1994, Trans. R. Soc. Trop. Med. Hyg., 88(4):491-3). Achieving this could help control the spread of the disease in endemic areas.

Euthanasia of dogs diagnosed with leishmaniasis is indeed a part of the official control measures endorsed by the Brazilian Ministry of Health to control Leishmaniasis in Brazil. The rationale behind this policy is based on public health concerns: dogs are considered the main reservoir for Leishmania infantum (which causes visceral leishmaniasis in humans) and controlling the disease in dogs is seen as a key step to prevent its spread to humans. However, this policy has been debated in terms of its effectiveness and ethical implications (Ministério da Saúde. Secretaria de Vigilância em Saúde.

Departamento de Vigilância Epidemiologica. Manual de vigilância e controle da leishmaniose visceral. Brasilia (DF); 2006).

Even taking out the role of other vertebrate hosts, data obtained by direct xenodiagnosis to test the infectivity of patients with a relative high parasite load of Leishmania infantum due to HIV/AIDS to the vector *Phlebotomus perniciosus* showed the direct transmission of Leishmania infantum from humans to the sand flies (Molina et al. 1999, Am. J. Trop. Med. Hyg., 60(1):51-53). Therefore, similarly to a vaccine designed for dogs, a potential vaccine for humans should lead to a decrease in the parasite load by promoting parasite clearance, apart from the primary goal of blocking the onset of disease symptoms in colonized humans.

Although several experimental trials in dogs have tested the effectiveness of vaccine candidates based on recombinant proteins, according to our data, none of the candidates ensured that 80% or more of the vaccinated and infected dogs became PCR-negative in the bone marrow (Tables 1 and 2). While the use of chemotherapy to treat the infection has shown some promising results, it is unable to completely clear the parasite and successfully eradicate the disease.

Therefore, there is currently a need for a vaccine that can effectively prevent and/or treat infections caused by parasites of the genus *Leishmania.* This vaccine should promote significant parasite clearance in a substantial proportion of the vaccinated and infected subjects, such as in 80% or more of dogs, specifically for preventing not only the disease but also the dissemination of canine leishmaniasis. Consequently, the objective of this application is to provide a combination product that can be used for the prevention and/or treatment of *Leishmania* infections. This product should be capable of reducing the parasite load in the bone marrow to levels undetectable by PCR in 80% or more of the vaccinated and infected dogs 64 weeks post-infection.

### Figures

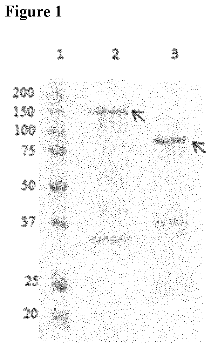

**Figure 1****:** 10% SDS-PAGE of purified AG1 and AG2. Lane 1: Molecular weight marker in kDa. Lane 2: PFR1-HSP70 (AG1). Lane 3: Q5 (AG2). Arrows show the band of the proteins of interest.

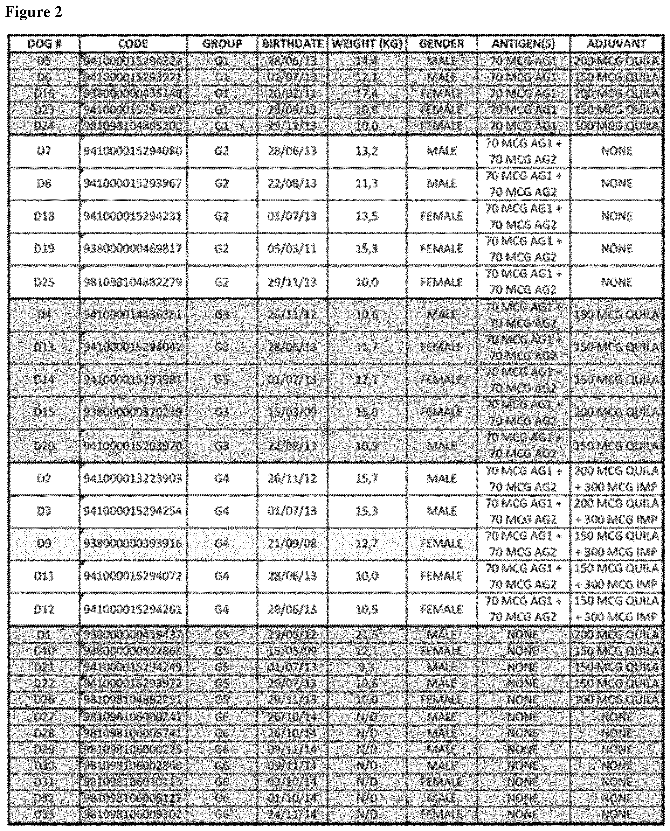

**Figure 2****:** Summary of dogs and immunogens used in the laboratory trial.

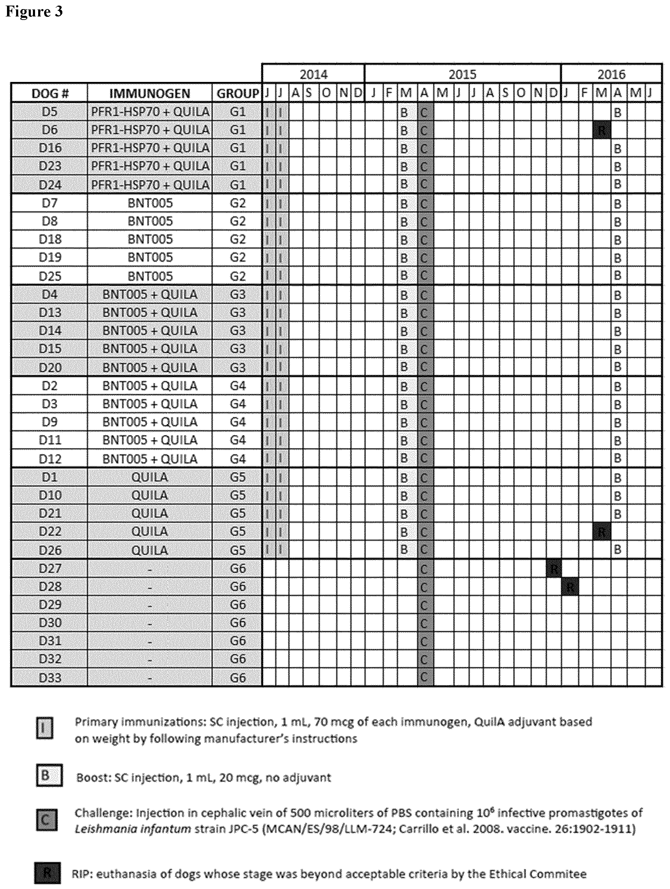

**Figure 3****:** Immunization schedule used in the laboratory trial.

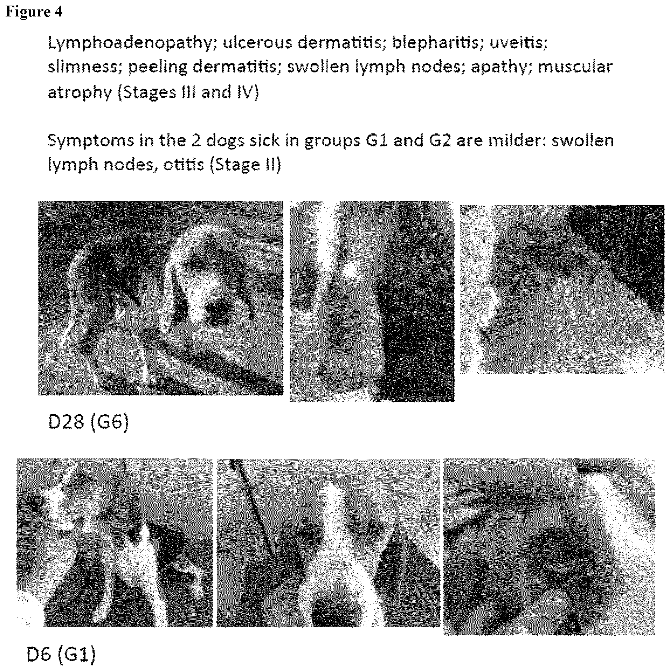

**Figure 4****:** Images of external symptoms in subject D28 and D6.

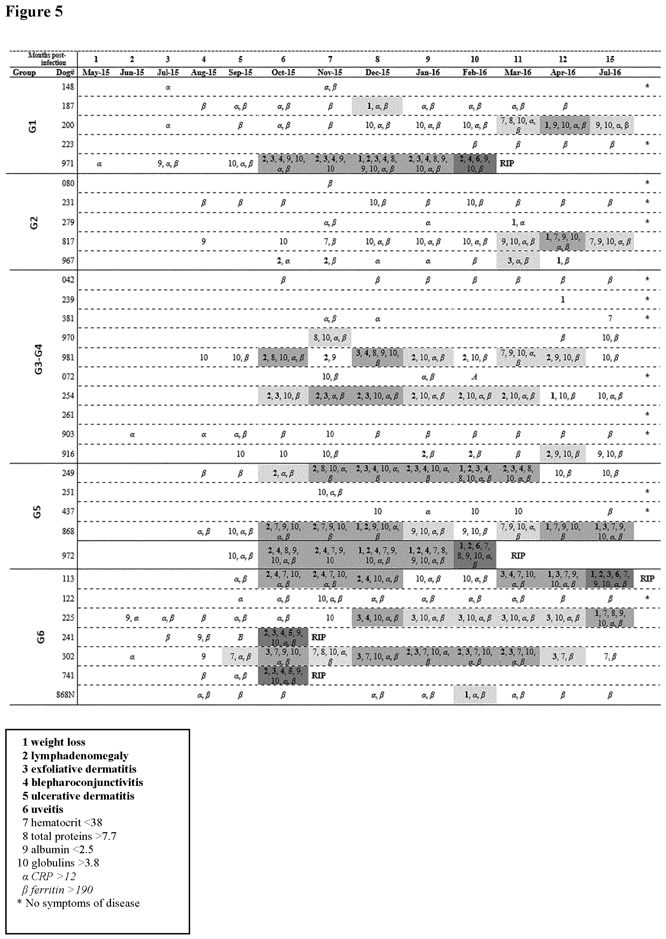

**Figure 5****:** Clinical and serological evolution of Beagle dogs after experimental infection with *Leishmania infantum* promastigotes. The legend shows several clinical symptoms (1 to 6), hematological & biochemical symptoms (7 to 10) or acute protein indicators related with the leishmaniasis disease. Grey scale color code indicates the health status of the dogs, from light to dark grey according to the severity. Rows marked with (*) correspond to dogs that did not show any symptom of disease along the study period.

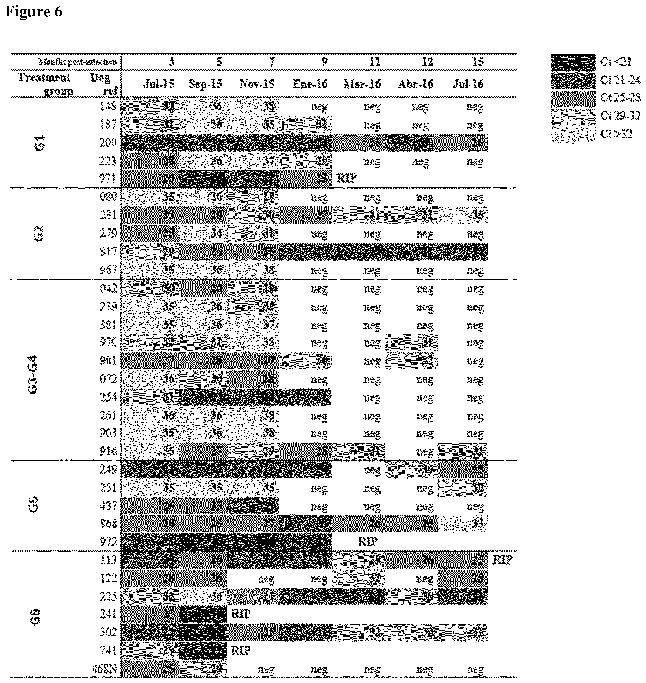

**Figure 6****:** Quantification of parasite load in the beagles. Parasite load is shown as Ct values. In this assay, Ct values above 38 represent undetectable levels of parasite. The Ct values are indicated in each cell unless the value is negative. In the color code, darker shades are correlated with higher parasite loads (smaller Ct-values).

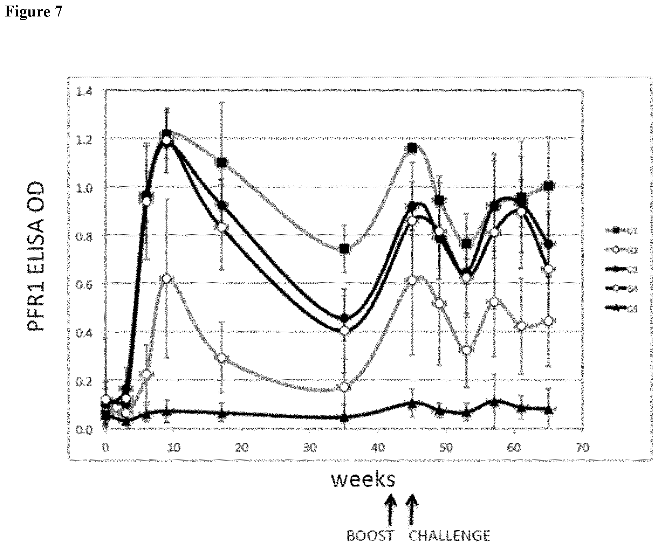

**Figure 7****:** Total IgG anti-PFR1 (AG1) in serum samples. OD values were measured in a direct ELISA by using *Leishmania infantum* PFR1 protein expressed and purified from E. *coli* as an antigen. Each data point represents an average value and the error bars represent ±1 standard deviation. Dogs in G1 were immunized with AG1 and Quil-A, in G2 were immunized with AG1 and AG2, in G3 were immunized with AG1, AG2 and Quil-A, in G4 were immunized with AG1, AG2, Quil-A and IMM-peptide, and in G5 were immunized with Quil-A.

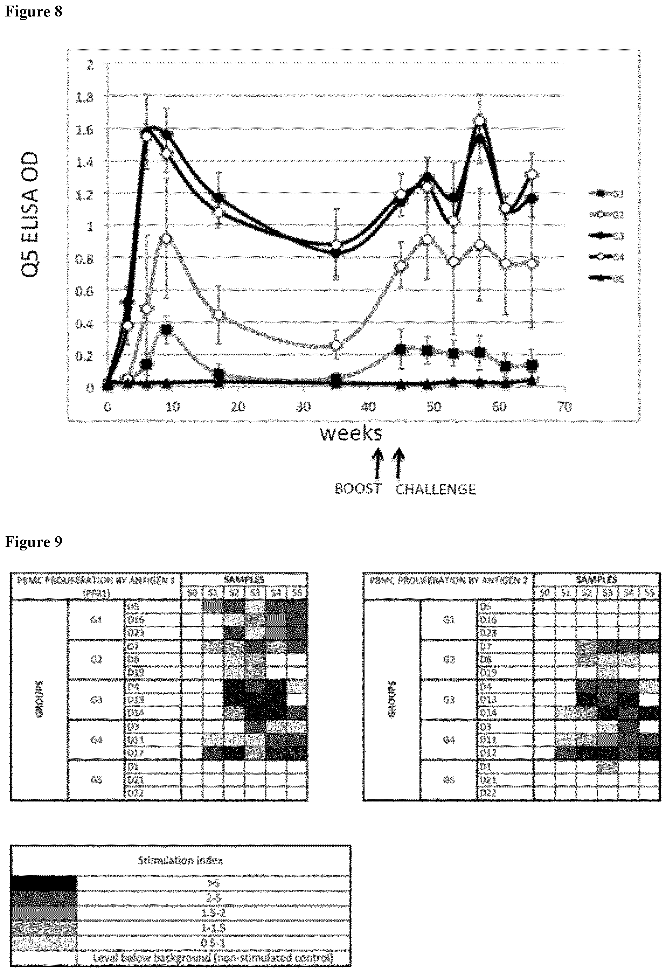

**Figure 8****:** Total IgG anti-AG2 in serum samples. OD values were measured in a direct ELISA by using AG2 protein expressed and purified from *E*. *coli* as an antigen. Each data point represents an average value and the error bars represent ±1 standard deviation. Dogs in G1 were immunized with AG1 and Quil-A, in G2 were immunized with AG1 and AG2, in G3 were immunized with AG1, AG2 and Quil-A, in G4 were immunized with AG1, AG2, Quil-A and IMM-peptide, and in G5 were immunized with Quil-A.
**Figure 9****:** Specific proliferation of peripheral blood mononuclear cells (PBMCs) from immunized dogs after stimulation with PFR1 (left panel) or AG2 (right panel). The cells were obtained from the dogs (D1-D23; 3 dogs per group G1 to G5) either before immunization (S0), 3 weeks after each immunization (S1, S2 and S3 respectively) or at 11 and 29 weeks after the last immunization (S4 and S5 respectively). The stimulation index was calculated as follows: s.i. = (radioactivity measured in stimulated cells - radioactivity measure in unstimulated cells) / radioactivity measured in unstimulated cells.

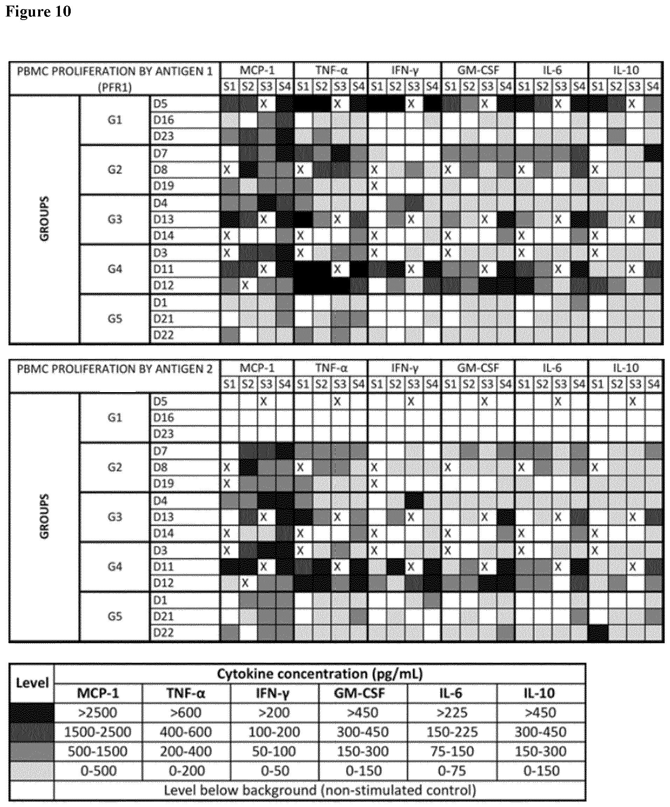

**Figure 10****:** Expression of cytokines by PBMCs stimulated by the antigens PFR1 (upper panel) or AG2 (lower panel). The cells were obtained from the dogs (D1-D23; 3 dogs per group G1 to G5) either before immunization (S0), 3 weeks after each immunization (S1, S2 and S3 respectively) or at 11 and 29 weeks after the last immunization (S4 and S5 respectively). The cell supernatant was harvested at either 24 h for determination of concentrations of cytokines TNF-α, IFN-γ, IL-6 and IL-10, or 48 h for determination of concentrations of cytokines MCP-1 and GM-CSF. An "X" in the panel means a sample value could not be determined because of technical problems.

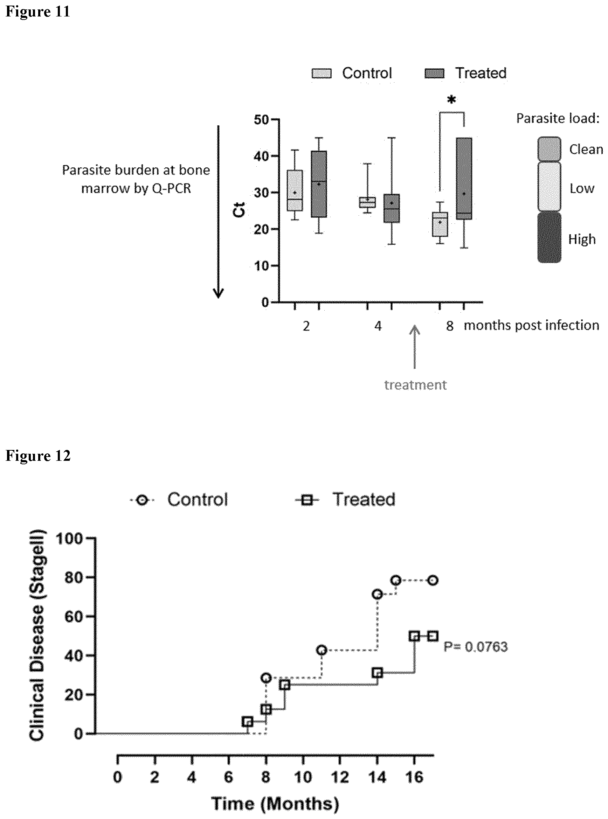

**Figure 11****:** Evolution of parasite burden (QPCR Ct values) at the bone marrow of dogs of the control and treated groups in the first year after experimental infection. Boxes represent interquartile range; horizontal line shows median Ct values; the cross, average Ct values; Error bars extend to the min and max value. Pairwise comparisons by 2-tailed Mann-Whitney test (*p<0.05). Lower Ct values are indicative of higher parasite load. A color code at the right of the figure shows the qualitative assessment of the quantitative Ct values according to calibration and use of the Q-PCR assay.
**Figure 12****:** Curve depicting the evolution of dogs reaching Stage 2 of canine leishmaniasis. Beagle dogs infected with 10⁶ *L. infantum* promastigotes by IV injection were monitored monthly for anti-SLA antibodies by ELISA and parasite load in the bone marrow by quantitative PCR. The dogs reaching a seropositive state and/or showing high parasite burden in the bone marrow were randomly distributed (1:1) into either the treated and control (untreated) group. Dogs in the treated group received 3 IM injections of BNT005 in 3-week intervals. Clinical symptoms and complete hematology and biochemistry analyses were done every 1-2 months in order to monitor the clinical evolution of each dog. The curves show the % of dogs reaching Stage 2 of canine leishmaniasis in both the treated and control groups over time (post infection).

### Summary of the invention

The present application provides, among other aspects and/or embodiments, a combination product, its use as a medicament, and its use as a medicament for the prevention and/or treatment of infections caused by parasites of the genus *Leishmania.* The combination product comprises (i) a first agent capable of leading to the antigenic presentation of a polypeptide as set forth in SEQ ID NO: 13 or a polypeptide which has at least 75 %, 80 %, 85 %, 90 %, 95 %, 96 %, 97 %, 98 %, or 99 % sequence identity with SEQ ID NO: 13, and (ii) a second agent capable of leading to the antigenic presentation of a polypeptide as set forth in SEQ ID NO: 14 or a polypeptide which has at least 75 %, 80 %, 85 %, 90 %, 95 %, 96 %, 97 %, 98 %, or 99 % sequence identity with SEQ ID NO: 14. The first and second agent can be administered together or separately. The first agent can be administered to an individual and/or animal who was already administered the second agent, and the second agent can be administered to an individual and/or animal who was already administered the first agent. Further, the combination product can be part of a kit for the prevention and/or treatment of infections caused by parasites of the genus *Leishmania.*

In an experimental trial, when the combination product was administered to seronegative dogs, which were later experimentally infected with the Leishmania parasite, the immune response exerted by the combination product in the dogs comprised a specific cytotoxic T-lymphocyte-mediated (CTL) response against the Leishmania antigens of the product and was able to promote parasite clearance in the bone marrow, defined as no parasites detectable by PCR in 90% of the vaccinated and infected dogs, 64 weeks after the infection (**Figure 6**). The parasite clearance correlated with the absence of clinical symptoms of Leishmaniasis in the vaccinated dogs 64 weeks after infection (**Figure 5**). In contrast, dogs immunized with only one of the two agents of the combination product, the chimeric protein PFR1-Hsp70, showed a negative PCR only in 60% of the of the vaccinated and infected dogs, 64 weeks after the infection (**Figure 6**) and, consistently, the dogs with a relatively high parasite load showed clinical symptoms of Leishmaniasis 64 weeks after infection (**Figure 5**).

In a different experimental trial, when the combination product was administered to seropositive and asymptomatic dogs, the immune response exerted by the product in the dogs was able to promote a significant (p<0.05) decrease of the mean parasite load detectable by PCR in the bone marrow, 35 weeks after the infection (**Figure 11**). Consistently, 15 months after infection of the dogs, and 10 months after treatment with the combination product, only 31% of the treated dogs showed clinical symptoms compatible with a Stage 2 of Leishmaniasis disease while 79% of the control untreated dogs reached this stage (**Figure 12**).

### TABLES 1 AND 2

**TABLE 1. Clinical symptoms**

| | **% Dogs with clinical symptoms** | | | |
|---|---|---|---|---|
| **Vaccine** | **Vaccinated** | **Control** | **Weeks after challenge** | **Reference** |
| H1 | 50% | 87.5% | 64 | Moreno et al. 2007 Vaccine 25: 5290-5300 |
| HASPB1 | 50% | 87.5% | 64 | Moreno et al. 2007 Vaccine 25: 5290-5300 |
| H1 + HASPB1 | 62.5% | 87.5% | 64 | Moreno et al. 2007 Vaccine 25: 5290-5300 |
| MML polyprotein | 71.4% | 87.5% | 64 | Moreno et al. 2007 Vaccine 25: 5290-5300 |
| Q polyprotein | 43% | 86% | 48 | Carcelén et al. 2009 Vaccine 27: 5964-5973 |
| A2 | 29% | 71% | 39 | Fernandes et al. 2008 Vaccine 26: 5888-5895 |
| CPA + CPB + IL12 | 100% | 100% | 56 | Poot et al. 2006 Vaccine 24: 2460-2468 |
| PFR1-HSP70 | 40% | 71% | 64 | This application |
| PFR1-HSP70 + Q5 | 0% | 71% | 64 | This application |

**TABLE 2. Parasite clearance**

| | **% PCR-negative dogs in bone marrow** | | | |
|---|---|---|---|---|
| **Vaccine (recombinant subunit vaccines)** | **Vaccinated** | **Control** | **Weeks after challenge** | **Reference** |
| H1 | 75% | 62.5% | 48 | Moreno et al. 2007 Vaccine 25: 5290-5300 |
| HASPB1 | 25% | 62.5% | 48 | Moreno et al. 2007 Vaccine 25: 5290-5300 |
| H1 + HASPB1 | 25% | 62.5% | 48 | Moreno et al. 2007 Vaccine 25: 5290-5300 |
| MML polyprotein | 43% | 62.5% | 48 | Moreno et al. 2007 Vaccine 25: 5290-5300 |
| Q polyprotein | 29% | 0% | 48 | Carcel6n et al. 2009 Vaccine 27: 5964-5973 |
| A2 | 71% | 29% | 39 | Fernandes et al. 2008 Vaccine 26: 5888-5895 |
| CPA + CPB + IL12 | 0% | 0% | 56 | Poot et al. 2006 Vaccine 24: 2460-2468 |
| LaPSA-38S | 78% | 0% | 26 | Petitdidier et al. 2016 PLOS Negl Trop Dis 10(5): e0004614 |
| LaPSA-12S | 80% | 0% | 26 | Petitdidier et al. 2016 PLOS Negl Trop Dis 10(5): e0004614 |
| A17G+A17E+E34PC | 60% | 0% | 26 | Petitdidier et al. 2019 npj Vaccines 4:49 |
| PFR1-HSP70 | 60% | 14% | 64 | This application |
| PFR1-HSP70 + Q5 | 90% | 14% | 64 | This application |

**TABLE 3. Clinical symptoms and parasite clearance in combination product.**

| % Dogs with clinical symptoms | | | |
|---|---|---|---|
| Vaccine | Vaccinated | Control | Weeks after challenge |
| ANTIGEN 1 | 40% | 71% | 64 |
| ANTIGEN 1 + ANTIGEN 2 | 0% | 71% | 64 |

| % PCR-negative dogs in bone marrow | | | |
|---|---|---|---|
| Vaccine | Vaccinated | Control | weeks after challenge |
| ANTIGEN 1 | 60% | 14% | 64 |
| ANTIGEN 1 + ANTIGEN 2 | 90% | 14% | 64 |

### Detailed description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

It is noted that the term "about", as used herein, refers to +/- 30%, +/- 20%, preferably +/- 15%, more preferably +/- 10%, of the indicated referred value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

"Percent (%) of sequence identity" with respect to proteins or polypeptides described herein is defined as the percentage of amino acid residues in a candidate sequence that are identical with the amino acid residues in the reference sequence (i.e., the protein or polypeptide from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared.

Preferably, the "percentage of identity" as used herein is decided in the context of a local alignment, i.e., it is based on the alignment of regions of local similarity between nucleobase sequences, contrary to a global alignment, which aims to align two sequences across their entire span. Thus, in the context of the present invention, percentage identity is calculated preferably only based on the local alignment comparison algorithm.

The term "*combination product*" can refer to (i) a product comprised of two or more regulated components that are physically, chemically, or otherwise combined or mixed and produced as a single entity; (ii) two or more separate products packaged together in a single package or as a unit and comprised of drug and device products, device and biological products, or biological and drug products; (iii) a drug, device, or biological product packaged separately that according to its investigational plan or proposed labeling is intended for use only with an approved individually specified drug, device, or biological product where both are required to achieve the intended use, indication, or effect and where upon approval of the proposed product the labeling of the approved product would need to be changed, e.g., to reflect a change in intended use, dosage form, strength, route of administration, or significant change in dose; or (iv) any investigational drug, device, or biological product packaged separately that according to its proposed labeling is for use only with another individually specified investigational drug, device, or biological product where both are required to achieve the intended use, indication, or effect.

The terms "*treatment*" and "*therapy*", as used in the present application, refer to a set of hygienic, pharmacological, surgical and/or physical means used with the intent to cure and/or alleviate a disease and/or symptoms with the goal of remediating the health problem. The terms "*treatment*" and "*therapy*" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term "*prevention*", as used in the present application, refers to a set of hygienic, pharmacological, surgical and/or physical means used to prevent the onset and/or development of a disease and/or symptoms. The term "*prevention*" encompasses prophylactic methods, since these are used to maintain the health of an animal or individual.

The term "*therapeutically effective amount*" refers to an amount of compound in a combination product which has a therapeutic effect and which is able to alleviate and/or cure a psychiatric and/or neurological disorder.

The terms "*individual*", "*patient*" or "*subject*" are used interchangeably in the present application and are not meant to be limiting in any way. The "*individual*", "*patient*" or "*subject*" can be of any age, sex and physical condition. The term "*animal*", as used in the present application, refers to any multicellular eukaryotic heterotroph which is not a human. Preferably, the animal is a dog.

The term "*vector*", as used in the present application, refers to a DNA or RNA molecule used as a vehicle to, optionally artificially, carry genetic material into a cell, where it can be replicated and/or expressed. A vector can also be used for DNA or RNA vaccination. The term "*DNA or RNA vaccination*" refers to a technique for treating and/or preventing a disease and/or infection with genetically engineered DNA or RNA. A vector can also be understood as any oligonucleotide encoding for any of the polypeptides described in the present invention.

The terms "*polypeptide*" or "*protein*" are used interchangeably in the present application and refer to a linear, branched and/or cyclic polymer consisting of at least 50 amino acid residues bonded together. The terms "*recombinant protein*" or "*recombinant polypeptide*", as used in the present application, refers to a protein encoded by a nucleic acid sequence that has been cloned in a system that supports expression of the nucleic acid sequence and translation of the mRNA.

The term "*vaccine*", as used in the present application, refers to both "*therapeutic vaccines*", which are intended to treat an existing disease and/or infection by strengthening the body's natural immune response, and "*prophylactic vaccines*", which are intended to prevent a disease and/or infection from developing in a healthy individual and/or animal.

In the present application, the term "variant" generally refers to a polypeptide sequence obtained through an amino acid modification (such as group substitution) or the insertion, substitution, and/or deletion of one or more amino acids (such as the deletion of a truncated form of an amino acid sequence) based on a natural polypeptide sequence. A variant may or may not retain the functions of the original natural polypeptide sequence in whole or in part and may also have improved functions compared with the original natural polypeptide sequence. A variant that retains the functions of the original natural polypeptide sequence in whole or in part or has improved functions compared with the original natural polypeptide sequence can be referred to as a functional variant. For example, the functional variant may exhibit a better biological activity (or function) than the original sequence. For example, an amino acid sequence of the functional variant may be at least 75%, 80%, 85%, 90%, 95%, 96%, 97%, 98%, or 99% identical to the original amino acid sequence.

### Combination product

The combination product BNT005 is composed by two chimeric molecules (Antigen 1 and Antigen 2), that are intended to be administered together, and in the presence of a saponin-type adjuvant, such as QuilA.

The Antigen 1 is composed by the fusion of the proteins, or the corresponding coding genes, PFR1 from *Leishmania infantum* (Li) and HSP70 from *Trypanosoma cruzi* (Tc). This antigen requires to be linked as a single fusion protein, and in the described arrangement. The Antigen 2 is composed by the fusion of the proteins, or the corresponding coding genes, KMP 11 of Li; polypeptide of the amino region of Li PSA protein; polypeptide of the carboxyl region of the Li PSA protein; polypeptide of the central region of the Li GP63 protein and polypeptide of the central region of the Tc HSP70 protein. These five polypeptides must be linked as a single fusion protein, and in the indicated arrangement.

To maintain the functionality as an effective vaccine against *Leishmania,* it is essential that the Antigen 1 preserve its native conformational structure obtained in prokaryotic or eukaryotic expression systems. PFR1 is a potent inducer of cellular and humoral immunity, and its immunological activity is enhanced by the association, as a fusion protein, with HSP70 antigen that shows immunomodulatory and dendritic cell maturation activity, thus leading to the levels necessary for effectiveness as vaccine.

In Antigen 2 fusion protein, the presence of all the polypeptides described is essential for the functionality as an effective vaccine against *Leishmania.* The KMP11 antigen, a highly conserved molecule among kinetoplastids, has a high immunogenic capacity, especially cellular immunity, and contains epitopes that induce a strong cytotoxic T lymphocyte response. The polypeptides of the amino and carboxyl regions of the PSA protein that are part of the Antigen 2, contain a potent T epitope (PSA_amino) and several B epitopes (PSA_carboxyl). The polypeptide of the GP63 protein, as part of the Antigen 2, contains a macrophage binding region. The polypeptide of HSP70 protein, contains the active ATP-binding regions and maintains, in the context of Antigen 2, the immunomodulatory and dendritic cell maturation activity of the complete HSP70 protein.

In a first aspect, the present application provides a combination product which comprises:
(i) a first agent capable of leading to the antigenic presentation of a polypeptide comprising or consisting of the amino acid sequences set forth in: SEQ ID NO: 1 or a functional variant thereof which has at least 75 %, 80 %, 85 %, 90 %, 95 %, 96 %, 97 %, 98 %, or 99 % sequence identity with SEQ ID NO: 1, and SEQ ID NO: 3 or a functional variant thereof which has at least 75 %, 80 %, 85 %, 90 %, 95 %, 96 %, 97 %, 98 %, or 99 % sequence identity with SEQ ID NO: 3, wherein both sequences are directly linked or optionally separated by a linker such as the linker set forth in SEQ ID NO: 2, and
(ii) a second agent capable of leading to the antigenic presentation of a polypeptide comprising or consisting of the amino acid sequences set forth in: SEQ ID NO: 4 or a functional variant thereof which has 75 %, 80 %, 85 %, 90 %, 95 %, 96 %, 97 %, 98 %, or 99 % sequence identity with SEQ ID NO: 4 directly linked, optionally through a linker such as the linker set forth in SEQ ID NO. 5, with SEQ ID NO: 6 or a functional variant thereof which has 75 %, 80 %, 85 %, 90 %, 95 %, 96 %, 97 %, 98 %, or 99 % sequence identity with SEQ ID NO: 6, which in turn is directly linked, optionally through a linker such as the linker set forth in SEQ ID NO: 7, with SEQ ID NO: 8 or a functional variant thereof which has 75 %, 80 %, 85 %, 90 %, 95 %, 96 %, 97 %, 98 %, or 99 % sequence identity with SEQ ID NO: 8, which in turn is directly linked, optionally through a linker such as the linker set forth in SEQ ID NO: 9, with SEQ ID NO: 10 or a functional variant thereof which has 75 %, 80 %, 85 %, 90 %, 95 %, 96 %, 97 %, 98 %, or 99 % sequence identity with SEQ ID NO: 10, which in turn is directly linked, optionally through a linker such as the linker set forth in SEQ ID NO: 11, with SEQ ID NO: 12 or a functional variant thereof which has 75 %, 80 %, 85 %, 90 %, 95 %, 96 %, 97 %, 98 %, or 99 % sequence identity with SEQ ID NO: 12.

In a preferred embodiment of the first aspect of the invention, the sequence of the polypeptide of the first agent has a sequence identity of at least 75%, 80%, 85%, 89%, 90%, 91%, 92%, 95%, 97%, 98% or 99% with SEQ ID NO: 13, preferably the sequence of the polypeptide of the first agent is SEQ ID NO: 13.

In another preferred embodiment of the first aspect of the invention, the sequence of the polypeptide of the second agent has a sequence identity of at least 75%, 80%, 85%, 89%, 90%, 91%, 92%, 95%, 97%, 98% or 99% with SEQ ID NO: 14, preferably the sequence of the polypeptide of the second agent is SEQ ID NO: 14.

In another preferred embodiment of the first aspect of the invention, the present application provides a combination product which comprises:
(i) a first agent capable of leading to the antigenic presentation of a polypeptide comprising or consisting of the amino acid sequence set forth in SEQ ID NO: 13 or a polypeptide which has at least 75 %, 80 %, 85 %, 90 %, 95 %, 96 %, 97 %, 98 %, or 99 % sequence identity with SEQ ID NO: 13, and
(ii) a second agent capable of leading to the antigenic presentation of a polypeptide comprising or consisting of the amino acid sequences set forth in SEQ ID NO: 14 or a polypeptide which has 75 %, 80 %, 85 %, 90 %, 95 %, 96 %, 97 %, 98 %, or 99 % sequence identity with SEQ ID NO: 14.

In another preferred embodiment of the first aspect, the present application provides a combination product which comprises:
(i) a first agent capable of leading to the antigenic presentation of a polypeptide comprising or consisting of the amino acid sequence set forth in SEQ ID NO: 13, and
(ii) a second agent capable of leading to the antigenic presentation of a polypeptide comprising or consisting of the amino acid sequences set forth in SEQ ID NO: 14.

SEQ ID NO: 1 corresponds to the amino acid sequence of PFR1 of Leishmania infantum. This sequence is, preferably via a linker such as SEQ ID NO: 2, fused or bound to HSP70 of *Trypanosoma cruzi* (SEQ ID NO: 3).
SEQ ID NO: 1 corresponds to the following sequence: 
PEDATGLEAARKQKIHNLKLKTACLENEELVQELHISDWSETQRQKLRGAHEKGEELLASVEVGTKWNLMEAYDLAKLMLVCG
LEMSQRELYRPEDKPQFMDIIGVKKVLQDLRQNRNKTRVVSFTQLIDNSIAKMEKVEEELRRSQLDATQLAQVPTRTVKMMED
IMNATQIQNALASTDDQMQTQLAQLEKTNEIQNVAMHDGEMQVAEEQMWTKVQLQERLIELLKGKFGLIGKCEEENAQFKEIY
EVQKQANQETSQMKDAKRRLRQRCETDLKHIQDAIQKADLEDAEAVKRYAGNKERSERAIKENEEMQEEAWNKIQDLERQLQN
LGTDRFDEVKRRIEEVDREEKRRVENAQFLEIAAQHKKLLELTVYNCDLAMRCTGLVEELVSEGCAGVKARYDKTNQDLAALR
LEVHKEHLEYFRMLYLTLGSLIYKKEKRLEEIDRNIRLAHIQLEFCVETFDPNAKKHADMKKELYRLRQGVEEELAMLKEKQA
AALDDFKESEEALDAAGIEFSHPVDENNEEVLTRRSKMVEYKSHLTKQEEVRIAAEREEIKRARLLRSGGESAAAQITSGSMN
ADYAASTQLEG
SEQ ID NO: 2
   YPKKTAGKKKE
SEQ ID NO: 3 
MTYEGAIGIDLGTTYSCVGVWQNERVEIIANDQGNRTTPSYVAFTDTERLIGDAAKNQVAMNPTNTVFDAKRLIGRKFSDPVV
QSDMKHWPFKVITKGDDKPVIQVQFRGETKTFNPEEVSSMVLSKMKEIAESYLGKQVKKAVVTVPAYFNDSQRQATKDAGTIA
GMEVLRIINEPTAAAIAYGLDKVEDGKERNVLIFDLGGGTFDVTLLTIDGGIFEVKATNGDTHLGGEDFDNRLVSHLTDEFKR
KNKGKDLTTSQRALRRLRTACERAKRTLSSAAQATIEIDALFDNVDFQATITRARFEELCGDLFRGTLQPVERVLQDAKMDKR
AVHDVVLVGGSTRIPKVMQLVSDFFGGKELNKSINPDEAVAYGAAVQAFILTGGKSKQTEGLLLLDVTPLTLGIETAGGVMTS
LIKRNTTIPTKKSQIFSTYADNQPGVHIQVFEGERAMTKDCHLLGTFDLSGIPPAPRGVPQIEVTFDLDANGILNVSAEEKGT
GKRNQIVIANDKGRLSKADIERMVSEAAKYEAQDKEQRERIDAKNGLENYAFSMKNTVNEPNVAGKIEEADKNTITSAVEEAL
QWLNNNQEASKEEYEHRQKELENLCTPIMTKMYQGMGAGGGMPGGMPGGMPGGMPGGMPGGMPGGMPGGMPGGMPGGMPGGMP
GGMPGGMPGGMPGGMPGGMPGGMPGGMPGGMPGGANPSSSSGPKVEEVD
SEQ ID NO: 4 corresponds to the amino acid sequence of KMP11 of Leishmania infantum. This sequence is fused or linked, preferably via a linker such as SEQ ID NO: 5, to the amino region of PSA of Leishmania infantum (SEQ ID NO: 6) which in turn is fused or linked, preferably via a linker such as SEQ ID NO: 7, to the carboxyl region of PSA of Leishmania infantum (SEQ ID NO: 8) which in turn is linked, preferably via a linker such as SEQ ID NO: 9, to gp63 of *Leishmania infantum* (SEQ ID NO: 10) which in turn is linked, preferably via a linker such as SEQ ID NO: 11, to HSP70 of *Trypanosoma cruzi* (SEQ ID NO: 12).
SEQ ID NO: 4 
MATTYEEFSAKLDRLDEEFNRKMQEQNAKFFADKPDESTLSPEMKEHYEKFERMIKEHTEKFNKKMHEHSEHFKQKFAELLEQ
QKAAQYPSK
SEQ ID NO: 5
   GACG
SEQ ID NO: 6 
AVKDDFTAAQRTNTLKVLQAFGRAIPELGKLWKGDDFCFWESVVCDVTEVYLWEIGATYTGTLPEMPVDVDYTAV
MVKHLDFSQMGLGLSGTLPASWSRLQGLTSLTLSG
SEQ ID NO: 7
   GTGG
SEQ ID NO: 8 
LKGNQFAGCVPDSWAQKAGLVVEIEDKHTGNSCIAGADCATTTTTTTEPTSTASPTATPTSAPETECEVDGCEVC
DGDSAARCARCREGYFLTDERTCL
SEQ ID NO: 9
   GGTG
SEQ ID NO: 10 
TTCQVFSDGHPAVGVINIPAANIASRYDQLVTRVVTHEMAHALGFSVGFFEGARILESISNVRHKDFDVPVINSSTAVAKARE
QYGCDTLEYLEIEDQGGAGSAGSHIKMRNAQDELMAPAAAAGYYSALTMAIFQDLGFYQADFSKAEVMPWGR
SEQ ID NO: 11
   GKLEF
SEQ ID NO: 12 
GKERNVLIFDLGGGTFDVTLLTIDGGIFEVKATNGDTHLGGEDFDNRLVSHLTDEFKRKNKGKDLTTSQRALRRLRTACERAK
RTLSSAAQATIEIDALFDNVDFQATITRARFEELCGDLFRGTLQPVERVLQDAKMDKRAVHDVVLVGGSTRIPKVMQLVSDFF
GGKELNKSINPDEAVAYGAAVQAFILTGGKSKQTEGLLLLDVTPLTLGIETAGGVMTSLIKRNTTIPTKKSQIFST
SEQ ID NO: 13 
MRGSHHHHHHGIPEDATGLEAARKQKIHNLKLKTACLENEELVQELHISDWSETQRQKLRGAHEKGEELLASVEVGTKWNLME
AYDLAKLMLVCGLEMSQRELYRPEDKPQFMDIIGVKKVLQDLRQNRNKTRVVSFTQLIDNSIAKMEKVEEELRRSQLDATQLA
QVPTRTVKMMEDIMNATQIQNALASTDDQMQTQLAQLEKTNEIQNVAMHDGEMQVAEEQMWTKVQLQERLIELLKGKFGLIGK
CEEENAQFKEIYEVQKQANQETSQMKDAKRRLRQRCETDLKHIQDAIQKADLEDAEAVKRYAGNKERSERAIKENEEMQEEAW
NKIQDLERQLQNLGTDRFDEVKRRIEEVDREEKRRVENAQFLEIAAQHKKLLELTVYNCDLAMRCTGLVEELVSEGCAGVKAR
YDKTNQDLAALRLEVHKEHLEYFRMLYLTLGSLIYKKEKRLEEIDRNIRLAHIQLEFCVETFDPNAKKHADMKKELYRLRQGV
EEELAMLKEKQAAALDDFKESEEALDAAGIEFSHPVDENNEEVLTRRSKMVEYKSHLTKQEEVRIAAEREEIKRARLLRSGGE
SAAAQITSGSMNADYAASTQLEGYPKKTAGKKKEMTYEGAIGIDLGTTYSCVGVWQNERVEIIANDQGNRTTPSYVAFTDTER
LIGDAAKNQVAMNPTNTVFDAKRLIGRKFSDPVVQSDMKHWPFKVITKGDDKPVIQVQFRGETKTFNPEEVSSMVLSKMKEIA
ESYLGKQVKKAVVTVPAYFNDSQRQATKDAGTIAGMEVLRIINEPTAAAIAYGLDKVEDGKERNVLIFDLGGGTFDVTLLTID
GGIFEVKATNGDTHLGGEDFDNRLVSHLTDEFKRKNKGKDLTTSQRALRRLRTACERAKRTLSSAAQATIEIDALFDNVDFQA
 
TITRARFEELCGDLFRGTLQPVERVLQDAKMDKRAVHDVVLVGGSTRIPKVMQLVSDFFGGKELNKSINPDEAVAYGAAVQAF
ILTGGKSKQTEGLLLLDVTPLTLGIETAGGVMTSLIKRNTTIPTKKSQIFSTYADNQPGVHIQVFEGERAMTKDCHLLGTFDL
SGIPPAPRGVPQIEVTFDLDANGILNVSAEEKGTGKRNQIVIANDKGRLSKADIERMVSEAAKYEAQDKEQRERIDAKNGLEN
YAFSMKNTVNEPNVAGKIEEADKNTITSAVEEALQWLNNNQEASKEEYEHRQKELENLCTPIMTKMYQGMGAGGGMPGGMPGG
MPGGMPGGMPGGMPGGMPGGMPGGMPGGMPGGMPGGMPGGMPGGMPGGMPGGMPGGMPGGMPGGANPSSSSGPKVEEVD
SEQ ID NO: 14

MRGSHHHHHHGSMATTYEEFSAKLDRLDEEFNRKMQEQNAKFFADKPDESTLSPEMKEHYEKFERMIKEHTEKFNKKMHEHSE
HFKQKFAELLEQQKAAQYPSKGACGAVKDDFTAAQRTNTLKVLQAFGRAIPELGKLWKGDDFCFWESVVCDVTEVYLWEIGAT
YTGTLPEMPVDVDYTAVMVKHLDFSQMGLGLSGTLPASWSRLQGLTSLTLSGGTGGLKGNQFAGCVPDSWAQKAGLVVEIEDK
HTGNSCIAGADCATTTTTTTEPTSTASPTATPTSAPETECEVDGCEVCDGDSAARCARCREGYFLTDERTCLGGTGTTCQVFS
DGHPAVGVINIPAANIASRYDQLVTRVVTHEMAHALGFSVGFFEGARILESISNVRHKDFDVPVINSSTAVAKAREQYGCDTL
EYLEIEDQGGAGSAGSHIKMRNAQDELMAPAAAAGYYSALTMAIFQDLGFYQADFSKAEVMPWGRGKLEFGKERNVLIFDLGG
GTFDVTLLTIDGGIFEVKATNGDTHLGGEDFDNRLVSHLTDEFKRKNKGKDLTTSQRALRRLRTACERAKRTLSSAAQATIEI
DALFDNVDFQATITRARFEELCGDLFRGTLQPVERVLQDAKMDKRAVHDVVLVGGSTRIPKVMQLVSDFFGGKELNKSINPDE
AVAYGAAVQAFILTGGKSKQTEGLLLLDVTPLTLGIETAGGVMTSLIKRNTTIPTKKSQIFST

It is noted that the amino acid sequence MRGSHHHHHHGI or MRGSHHHHHHGS is a purification tag that can be present or not in any of sequences 13 or 14 and that can be replaced by any other purification tags. That is, SEQ ID NO 13 and 14 may contain or completely lack these sequences as represented below in SEQ ID NO 15 or 16:
SEQ ID NO: 15 
MPEDATGLEAARKQKIHNLKLKTACLENEELVQELHISDWSETQRQKLRGAHEKGEELLASVEVGTKWNLMEAYDLAKLMLVC
GLEMSQRELYRPEDKPQFMDIIGVKKVLQDLRQNRNKTRVVSFTQLIDNSIAKMEKVEEELRRSQLDATQLAQVPTRTVKMME
DIMNATQIQNALASTDDQMQTQLAQLEKTNEIQNVAMHDGEMQVAEEQMWTKVQLQERLIELLKGKFGLIGKCEEENAQFKEI
YEVQKQANQETSQMKDAKRRLRQRCETDLKHIQDAIQKADLEDAEAVKRYAGNKERSERAIKENEEMQEEAWNKIQDLERQLQ
NLGTDRFDEVKRRIEEVDREEKRRVENAQFLEIAAQHKKLLELTVYNCDLAMRCTGLVEELVSEGCAGVKARYDKTNQDLAAL
RLEVHKEHLEYFRMLYLTLGSLIYKKEKRLEEIDRNIRLAHIQLEFCVETFDPNAKKHADMKKELYRLRQGVEEELAMLKEKQ
AAALDDFKESEEALDAAGIEFSHPVDENNEEVLTRRSKMVEYKSHLTKQEEVRIAAEREEIKRARLLRSGGESAAAQITSGSM
NADYAASTQLEGYPKKTAGKKKEMTYEGAIGIDLGTTYSCVGVWQNERVEIIANDQGNRTTPSYVAFTDTERLIGDAAKNQVA
MNPTNTVFDAKRLIGRKFSDPVVQSDMKHWPFKVITKGDDKPVIQVQFRGETKTFNPEEVSSMVLSKMKEIAESYLGKQVKKA
VVTVPAYFNDSQRQATKDAGTIAGMEVLRIINEPTAAAIAYGLDKVEDGKERNVLIFDLGGGTFDVTLLTIDGGIFEVKATNG
DTHLGGEDFDNRLVSHLTDEFKRKNKGKDLTTSQRALRRLRTACERAKRTLSSAAQATIEIDALFDNVDFQATITRARFEELC
GDLFRGTLQPVERVLQDAKMDKRAVHDVVLVGGSTRIPKVMQLVSDFFGGKELNKSINPDEAVAYGAAVQAFILTGGKSKQTE
GLLLLDVTPLTLGIETAGGVMTSLIKRNTTIPTKKSQIFSTYADNQPGVHIQVFEGERAMTKDCHLLGTFDLSGIPPAPRGVP
QIEVTFDLDANGILNVSAEEKGTGKRNQIVIANDKGRLSKADIERMVSEAAKYEAQDKEQRERIDAKNGLENYAFSMKNTVNE
PNVAGKIEEADKNTITSAVEEALQWLNNNQEASKEEYEHRQKELENLCTPIMTKMYQGMAGGGMPGGMPGGMPGGMPGGMPG
GMPGGMPGGMPGGMPGGMPGGMPGGMPGGMPGGMPGGMPGGMPGGMPGGMPGGANPSSSSGPKVEEVD
SEQ ID NO: 16

MATTYEEFSAKLDRLDEEFNRKMQEQNAKFFADKPDESTLSPEMKEHYEKFERMIKEHTEKFNKKMHEHSEHFKQKFAELLEQ

QKAAQYPSKGACGAVKDDFTAAQRTNTLKVLQAFGRAIPELGKLWKGDDFCFWESVVCDVTEVYLWEIGATYTGTLPEMPVDV

DYTAVMVKHLDFSQMGLGLSGTLPASWSRLQGLTSLTLSGGTGGLKGNQFAGCVPDSWAQKAGLVVEIEDKHTGNSCIAGADC

ATTTTTTTEPTSTASPTATPTSAPETECEVDGCEVCDGDSAARCARCREGYFLTDERTCLGGTGTTCQVFSDGHPAVGVINIP
AANIASRYDQLVTRVVTHEMAHALGFSVGFFEGARILESISNVRHKDFDVPVINSSTAVAKAREQYGCDTLEYLEIEDQGGAG
SAGSHIKMRNAQDELMAPAAAAGYYSALTMAIFQDLGFYQADFSKAEVMPWGRGKLEFGKERNVLIFDLGGGTFDVTLLTIDG
GIFEVKATNGDTHLGGEDFDNRLVSHLTDEFKRKNKGKDLTTSQRALRRLRTACERAKRTLSSAAQATIEIDALFDNVDFQAT
ITRARFEELCGDLFRGTLQPVERVLQDAKMDKRAVHDVVLVGGSTRIPKVMQLVSDFFGGKELNKSINPDEAVAYGAAVQAFI
LTGGKSKQTEGLLLLDVTPLTLGIETAGGVMTSLIKRNTTIPTKKSQIFST

It is further noted that the term "linker", as used herein, shall not limit the present invention as any peptide linkers that preserve the native conformational structure obtained in prokaryotic or eukaryotic expression systems of any of Antigens 1 or 2 shall be suitable. Examples of suitable linkers include, without limitation, Glycine-Serine (Gly-Ser) linkers {e.g.: (Gly-Gly-Gly-Gly-Ser)n, wherein n can vary from 1 to 5}; Alanine-Glycine (Ala-Gly) linkers {e.g.: (Ala-Ala-Ala-Gly-Gly)n, wherein n can vary from 1 to 5}; and Glycine-Serine-Threonine (Gly-Ser-Thr) linkers {e.g.: (Gly-Ser-Thr)n, wherein n can vary from 1 to 8}. All of these examples of linkers constitute, in an embodiment of the present invention, potential replacements of any of the specific linkers identified in the previous embodiments of the invention.

The term "*capable of leading to the antigenic presentation of a polypeptide*" refers to the ability of an agent to elicit an immune response in an animal or individual which is specific for a given antigen, i.e. the polypeptide. This can be achieved by both administering an oligonucleotide, such as a DNA or RNA vaccine, that encodes for the polypeptide and/or by administering a polypeptide or recombinant protein which comprises the polypeptide. In one embodiment, the agent capable of leading to the antigenic presentation of a polypeptide is a DNA or RNA vaccine which encodes the polypeptide. The DNA or RNA vaccine is administered to an individual or animal and is then incorporated into a host cell where the polypeptide is expressed and then its antigens are presented to the immune system. In another embodiment, the agent capable of leading to the antigenic presentation of a polypeptide is a, preferably recombinant, protein or polypeptide.

It is herein noted by "oligonucleotide," as referred herein is meant any short segment of DNA, RNA, or DNA/RNA, including both natural and synthetic nucleotides. As used in this invention, the term "oligonucleotide " includes both oligonucleotides as such, as well as the "oligonucleotide analogues". "Oligonucleotide analogues" are the molecules derived therefrom that incorporate some chemical modification in at least one of the nucleotide units that form them, either in the phosphate group, the pentose or one of the nitrogenous bases.

It is herein noted that in an embodiment of the first aspect of the present invention, the first agent capable of leading to the antigenic presentation is a fusion polypeptide as defined in the first aspect of the invention. That is, the first agent is characterized by comprising or consisting of SEQ ID NO 1 and SEQ ID NO 3, or any functional variants thereof, wherein the polypeptides are linked directly or optionally through linkers, in the indicated arrangement order, that is SEQ ID NO 1 is linked directly, optionally through a linker, to SEQ ID NO 3.

It is herein further noted that in an embodiment of the first aspect of the present invention, the second agent capable of leading to the antigenic presentation is a fusion polypeptide (from herein after referred to as the second polypeptide agent of the invention) as defined in the first aspect of the inventon. That is, the second agent is characterized by comprising or consisting of SEQ ID NO 4, SEQ ID NO 6, SEQ ID NO 8, SEQ ID NO 10 and SEQ ID NO 12, or any functional variants thereof, wherein the polypeptides are linked directly or optionally through linkers, in the indicated arrangement order, that is SEQ ID NO 4 is linked directly, or optionally through a linker, to SEQ ID NO 6, which in turn is linked directly, or optionally through a linker, to SEQ ID NO 8, which in turn is linked directly, or optionally through a linker, to SEQ ID NO 10, which in turn is linked directly, or optionally through a linker, to SEQ ID NO 12.

It is herein further noted that in another embodiment of the first aspect of the present invention, the first agent capable of leading to the antigenic presentation is an oligonucleotide sequence encoding for the first polypeptide agent of the invention (from herein after referred to as the oligonucleotide encoding for the first polypeptide agent of the invention).

It is herein further noted that in another embodiment of the first aspect of the present invention, the second agent capable of leading to the antigenic presentation is an oligonucleotide sequence encoding for the second polypeptide agent of the invention (from herein after referred to as the oligonucleotide encoding for the second polypeptide agent of the invention).

In another embodiment of the first aspect of the invention, optionally in combination with any of the previous or subsequent embodiments of the first aspect of the invention, the first and second agents capable of leading to the antigenic presentation are, respectively, the oligonucleotide encoding for the first polypeptide agent of the invention and the oligonucleotide encoding for the second polypeptide agent of the invention, wherein both are contained in one vector capable of leading to the antigenic presentation of both polypeptides, or in an alternative embodiment, both are contained in two vectors capable of leading to the antigenic presentation of both polypeptides.

A number of methods are conveniently used to deliver nucleic acids to a patient. For instance, nucleic acid can be delivered directly, as "*naked DNA*" or as "naked RNA". This approach is described, for instance, in Wolff, et al, Science 247: 1465-1468 (1990) as well as U.S. Patent Nos. 5,580,859 and 5,589,466. Nucleic acids can also be administered using ballistic delivery as described, for instance, in U.S. Patent No. 5,204,253. Particles comprised solely of DNA and/or RNA can be administered.

Alternatively, DNA and/or RNA can be adhered to particles, such as gold particles. The nucleic acids can also be delivered complexed to cationic compounds, such as cationic lipids. Lipid-mediated gene delivery methods are described, for instance, in WO 96/18372; WO 93/24640; Mannino & Gould-Fogerite, BioTechniques 6(7): 682-691 (1988); Rose U.S. Pat No. 5,279,833; WO 91/06309; and Feigner, et al, Proc. Natl. Acad. Sd. USA 84: 7413-7414 (1987).

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted above, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

In a preferred embodiment, the polypeptide encoded by the oligonucleotide encoding for the first polypeptide agent of the invention is set forth in SEQ ID NO: 13 or a sequence which has at least 75 % sequence identity with SEQ ID NO: 13. Preferably, the polypeptide encoded by the oligonucleotide encoding for the first polypeptide agent of the invention has a sequence identity of at least 75%, 80%, 85%, 89%, 90%, 91%, 92%, 95%, 97%, 98% or 99% with SEQ ID NO: 13. In a preferred embodiment, the polypeptide encoded by the oligonucleotide encoding for the second polypeptide agent of the invention is set forth in SEQ ID NO: 14 or a sequence which has at least 75 % sequence identity with SEQ ID NO: 14. Preferably, the polypeptide encoded by the oligonucleotide encoding for the second polypeptide agent of the invention has a sequence identity of at least 75%, 80%, 85%, 89%, 90%, 91%, 92%, 95%, 97%, 98% or 99% with SEQ ID NO: 14.

In a preferred embodiment, the first polypeptide agent of the invention is a recombinant protein. In a preferred embodiment, the second polypeptide agent of the invention is a recombinant protein.

The first and second polypeptide agents of the invention may be separate in solution or bound and/or complexed to one another. The first and second polypeptide agents of the invention may be expressed as a single fusion protein or as two separate proteins. The first and second polypeptide agents of the invention may be expressed as two separate proteins which may then be conjugated together through covalent and/or non-covalent interactions. For example, the first and second polypeptide agents of the invention may be biotinylated ad conjugated using streptavidin, the first and second polypeptide agents of the invention may be fused together using a split intein or isopeptide-mediated approaches such as the SpyTag/SpyCatcher system, or the first and second polypeptide agents of the invention may be conjugated to one another using amine-reactive crosslinker chemistry or sulfhydryl-reactive crosslinker chemistry.

In addition to the recombinant proteins, a combination product as described herein may also contain other substances. These substances include, but are not limited to, cryoprotectants, lyoprotectants, surfactants, bulking agents, anti-oxidants, and stabilizing agents. In some embodiments, the combination product may be lyophilized.

The term "*cryoprotectant*" as used herein, includes agents which provide stability to the protein against freezing-induced stresses, by being preferentially excluded from the protein surface. Cryoprotectants may also offer protection during primary and secondary drying and long-term product storage. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In one embodiment, a lyoprotectant is added to a combination product described herein. The term "*lyoprotectant*" as used herein, includes agents that provide stability to the protein during the freeze-drying or dehydration process (primary and secondary freeze- drying cycles), by providing an amorphous glassy matrix and by binding with the protein through hydrogen bonding, replacing the water molecules that are removed during the drying process. This helps to maintain the protein conformation, minimize protein degradation during the lyophilization cycle, and improve the long-term product stability. Non-limiting examples of lyoprotectants include sugars, such as sucrose or trehalose; an amino acid, such as monosodium glutamate, non-crystalline glycine or histidine; a methylamine, such as betaine; a lyotropic salt, such as magnesium sulfate; a polyol, such as trihydric or higher sugar alcohols, e.g., glycerin, erythritol, glycerol, arabitol, xylitol, sorbitol, and mannitol; propylene glycol; polyethylene glycol; pluronics; and combinations thereof. The amount of lyoprotectant added to a combination product is generally an amount that does not lead to an unacceptable amount of degradation/aggregation of the protein when the combination product is lyophilized.

In certain embodiments, a surfactant may be included in the combination product. The term "*surfactant*" as used herein, includes agents that reduce the surface tension of a liquid by adsorption at the air-liquid interface. Examples of surfactants include, without limitation, nonionic surfactants, such as polysorbates {e.g., polysorbate 80 or polysorbate 20); poloxamers (e.g., poloxamer 188); Triton(TM); sodium dodecyl sulfate (SDS); sodium laurel sulfate; sodium octyl glycoside; lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl- betaine, cetyl-betaine, lauroamidopropyl-betaine, cocamidopropyl-betaine, linoleamidopropyl-betaine, myristamidopropyl-betaine, palmidopropyl-betaine, isostearamidopropyl-betaine (e.g., lauroamidopropyl), myristamidopropyl-, palmidopropyl-, or isostearamidopropyl- dimethylamine; sodium methyl cocoyl-, or disodium methyl ofeyl-taurate; and the Monaquat(TM) series (Mona Industries, Inc., Paterson, NJ.), polyethyl glycol, polypropyl glycol, and copolymers of ethylene and propylene glycol (e.g., pluronics, PF68). The amount of surfactant added is such that it maintains aggregation of the reconstituted protein at an acceptable level as assayed using, e.g., SEC-HPLC to determine the percentage of HMW species or LMW species, and minimizes the formation of particulates after reconstitution of a lyophilate of a protein.

In some embodiments, a bulking agent is included in the combination product. The term "*bulking agent*" as used herein, includes agents that provide the structure of the freeze- dried product without interacting directly with the pharmaceutical product. In addition to providing a pharmaceutically elegant cake, bulking agents may also impart useful qualities in regard to modifying the collapse temperature, providing freeze-thaw protection, and enhancing the protein stability over long-term storage. Non-limiting examples of bulking agents include mannitol, glycine, lactose, and sucrose. Bulking agents may be crystalline (such as glycine, mannitol, or sodium chloride) or amorphous (such as dextran, hydroxyethyl starch) and are generally used in protein formulations in an amount from 0.5% to 10%.

Other pharmaceutically acceptable carriers, excipients, or stabilizers, such as those described in Remington's Pharmaceutical Sciences 16th edition, Osol, A. Ed. (1980) may also be included in a combination product described herein, provided that they do not adversely affect the desired characteristics of the combination product. As used herein, "*pharmaceutically acceptable carrier*" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

In a preferred embodiment, both the first and second polypeptide agents of the invention are recombinant proteins. In an alternative embodiment, the first and second agents capable of leading to the antigenic presentation are oligonucleotide sequences encoding for the first and second polypeptide agents of the invention, which might optionally be contained in one or two vectors for DNA and/or RNA vaccination. In one embodiment, the first agent capable of leading to the antigenic presentation is a recombinant protein and the second agent capable of leading to the antigenic presentation is a nucleotide sequence contained in one vector for DNA and/or RNA vaccination. In another embodiment, the second agent capable of leading to the antigenic presentation is a recombinant protein and the first agent capable of leading to the antigenic presentation is a nucleotide sequence contained in one vector for DNA and/or RNA vaccination. In a preferred embodiment, the combination product may also be lyophilized.

In a preferred embodiment, the combination product further comprises an adjuvant. Preferably, the adjuvant is selected from the list consisting of Alum, Freund's Incomplete Adjuvant, MF59^{®}, synthetic analogs of dsRNA such as poly(I:C), bacterial lipopolysaccharides, bacterial flagellin, imidazolquinolines, oligodeoxynucleotides containing specific CpG motifs, fragments of bacterial cell walls such as muramyl dipeptide, TLR adjuvants like TLRL-2 (Pam3CSK4) and TLRL-3 (Poly(I:C) HMW and adjuvants based on saponins like Quil-A^{®} or purified fractions from Quil-A like QS21. Preferably, the adjuvant is Quil-A^{®}. In another embodiment, the combination product does not comprise an adjuvant.

In a preferred embodiment, the combination product is a mixture of the first agent and the second agent, i.e. the combination product is a composition. In an alternative embodiment, the first agent and second agent are physically separated. For example, the first agent could be contained in one vial while the second agent could be contained in a separate vial.

In a preferred embodiment, the combination product comprises the first agent and instructions on how to administer the first agent with the second agent. In another preferred embodiment, the combination product comprises the second agent and instructions on how to administer the second agent with the first agent.

The combination product may be prepared for oral, sublingual, buccal, intravenous, intramuscular, subcutaneous, rectal, transdermal, topical and/or inhalation-mediated administration. In a preferred embodiment, the combination product may comprise one or more solution(s) which are suitable for intravenous, intramuscular, transdermal and/or subcutaneous administration. In another embodiment, the combination product may comprise one or more solution(s) which are suitable for sublingual, buccal and/or inhlation-mediated administration routes. In an alternative embodiment, the combination product may comprise one or more aerosol(s) which are suitable for inhlation-mediated administration.

In a preferred embodiment, the combination product may comprise one or more cream(s) and/or ointment(s) which are suitable for topical administration. In a preferred embodiment, the combination product may comprise one or more suppositories which are suitable for rectal administration.

The combination product may also be administered via liposomes. Liposomes include emulsions, foams, micelles, insoluble monolayers, liquid crystals, phospholipid dispersions, lamellar layers and the like. In these preparations the polypeptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic combination products. Thus, liposomes either filled or decorated with a desired polypeptide can be directed to the site of lymphoid cells, where the liposomes then deliver the selected therapeutic/immunogenic polypeptide combination products. Liposomes are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka, et al., Ann. Rev. Biophys. Bioeng. 9:467 (1980), U.S. Patent Nos. 4,235,871, 4,501,728, 4,837,028, and 5,019,369.

The combination product may further comprise common excipients and carriers which are known in the state of the art. For solid combination products, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For solution for injection, the combination product may further comprise cryoprotectants, lyoprotectants, surfactants, bulking agents, anti-oxidants, stabilizing agents and pharmaceutically acceptable carriers. For aerosol administration, the combination products are generally supplied in finely divided form along with a surfactant and propellant. The surfactant must, of course, be nontoxic, and is generally soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. For suppositories, traditional binders and carriers may include, for example, polyalkalene glycols or triglycerides.

The combination product may comprise any combination of tablets, solutions, aerosols, creams, ointments and/or suppositories as long as the combination product is suitable for the treatment and/or prevention of infections caused by parasites of the genus *Leishmania.*

In a preferred embodiment, the combination product is administered at least once, preferably at least two times, or more than two times. A dosage of the combination product can comprise 0.5 - 1000 mcg of the first agent and/or 0.5 - 1000 mcg of the second agent. Preferably, an optimal dosage of the combination product can comprise 70 mcg of the first agent and/or 70 mcg of the second agent.

In a preferred embodiment the combination product is a vaccine capable of inducing an immune response. The design of combination products for vaccines is well established, and is described, for example, in Remington's Pharmaceutical Sciences, latest edition, Mack Publishing Co., Easton, PA, and in Plotkin and Orenstein's book entitled Vaccines, 4th Ed., Saunders, Philadelphia, PA (2004). Preferably the immune response is a humoral immune response, a CD8+ T cell-mediated immune response and/or a CD4+ T cell-mediated immune response against the *Leishmania* antigens PFR1, KMP11, PSA and gp63. The humoral immune response might be monitored by detection of total IgG and IgG subtypes in the sera of the immunized subjects by using techniques like ELISA with plates coated with the antigens of the vaccine. The cellular response might be monitored by using primary cultures of peripheral blood mononuclear cells (PBMCs) taken from the vaccinated subjects and measuring their stimulation by Leishmania antigens. The nature of the stimulated cells could be measured by detecting them with specific antibodies by using flow cytometry. The release of specific cytokines markers of a CD8+ mediated or a CD4+ mediated cellular response can be detected in the supernatant of the stimulated cells by different available analytical techniques, including ELISPOT.

### Uses of the combination product

In a second aspect, the combination product of the present invention can be used as a medicament. In a third aspect, the combination product of the present invention can be used for the treatment and/or prevention of infections caused by parasites of the genus *Leishmania.*

The present invention also encompasses the use of a combination product as defined in the first aspect or in any of its preferred embodiments, for the treatment and/or prevention of infections caused by parasites of the genus *Leishmania.*

The present invention also encompasses a method for the treatment and/or prevention of infections caused by parasites of the genus *Leishmania* which comprises administering a therapeutically effective amount of (i) a first agent capable of leading to the antigenic presentation of a polypeptide as set forth in the first aspect or in any of its preferred embodiments, and (ii) a second agent capable of leading to the antigenic presentation of a polypeptide as set forth in in the first aspect or in any of its preferred embodiments to an individual or animal who suffers or is at risk of suffering an infection.

In a preferred embodiment, the parasites of the genus *Leishmania* are selected from *Leishmania infantum, Leishmania donovani, Leishmania chagasi, Leishmania tropica, Leishmania major, Leishmania braziliensis, or Leishmania mexicana.* Preferably, the parasites of the genus *Leishmania* is of the species *Leishmania infantum.*

The combination product may be administered to animals and humans. In a preferred embodiment, the combination product is administered to an individual who suffers or is at risk of suffering an infection caused by a parasite of the genus *Leishmania.* In another embodiment, the combination product is administered to a dog who suffers or is at risk of suffering an infection caused by a parasite of the genus *Leishmania.*

### Kit

In a fourth aspect, the combination product of the present invention is part of a kit for the treatment and/or prevention of infections caused by parasites of the genus *Leishmania.*

In a preferred embodiment, the parasites of the genus *Leishmania* are selected from *Leishmania infantum, Leishmania donovani, Leishmania chagasi, Leishmania tropica, Leishmania major, Leishmania braziliensis, or Leishmania mexicana.* Preferably, the parasites of the genus *Leishmania* is of the species *Leishmania infantum.*

### Administration

The first agent and second agent may be administered together or separately to an animal or individual who suffers or is at risk of suffering an infection caused by a parasite of the genus *Leishmania.*

In a preferred embodiment, the first agent may be administered to an animal or individual who is already administered the second agent and who suffers or is at risk of suffering an infection caused by a parasite of the genus *Leishmania.* In an alternative embodiment, the second agent may be administered to an animal or individual who is already administered the first agent and who suffers or is at risk of suffering an infection caused by a parasite of the genus *Leishmania.*

In a preferred embodiment, the administration of the combination product to an animal or individual who suffers or is at risk of suffering an infection caused by a parasite of the genus *Leishmania* leads to the combination product inducing an immune response. Preferably the immune response is a humoral immune response, a CD8+ T cell-mediated immune response and/or a CD4+ T cell-mediated immune response against the *Leishmania* antigens PFR1, KMP 11, PSA and gp63. The humoral immune response might be monitored by detection of total IgG and IgG subtypes in the sera of the immunized subjects by using techniques like ELISA with plates coated with the antigens of the vaccine. The cellular response might be monitored by using primary cultures of peripheral blood mononuclear cells (PBMCs) taken from the vaccinated subjects and measuring their stimulation by Leishmania antigens. The nature of the stimulated cells could be measured by detecting them with specific antibodies by using flow cytometry. The release of specific cytokines markers of a CD8+ mediated or a CD4+ mediated cellular response can be detected in the supernatant of the stimulated cells by different available analytical techniques, including ELISPOT.

In a preferred embodiment, the combination product is administered to prevent the onset of disease associated with infections caused by a parasite of the genus *Leishmania.* For example, the combination product can be administered to an animal or individual who is at risk of developing an infection caused by a parasite of the genus *Leishmania.*

In a preferred embodiment, the combination product is administered to treat an infection caused by a parasite of the genus *Leishmania.* For example, the combination product can be administered to an animal or individual who suffers from an infection caused by a parasite of the genus *Leishmania.*

In a preferred embodiment, the combination product is administered to lead to an estate of low parasite burden in an animal or individual infected by parasites of the genus Leishmania either previously or after the administration of the combination product, with the goal of reducing the risk of transmission of the parasite to the Phlebotominae vector.

In a preferred embodiment, the combination product is prepared for oral, sublingual, buccal, intravenous, intramuscular, subcutaneous, rectal, transdermal, topical and/or inhalation-mediated administration routes, preferably intravenous, intramuscular, subcutaneous and/or transdermal routes.

In a preferred embodiment, the combination product is administered once every year.

Although several experimental trials in dogs have tested the effectiveness of vaccine candidates based on recombinant proteins, according to our data, none of the candidates ensured that 80% or more of the vaccinated and infected dogs became PCR-negative in the bone marrow (Tables 1 and 2).

Therefore, there is currently a need for a vaccine that can effectively prevent and/or treat infections caused by parasites of the genus *Leishmania.* This vaccine should promote significant parasite clearance in a substantial proportion of the vaccinated and infected subjects, such as in 80% or more of dogs, specifically for preventing not only the disease but also the dissemination of canine leishmaniasis.

The present application provides a combination product, its use as a medicament, and its use as a medicament for the prevention and/or treatment of infections caused by parasites of the genus *Leishmania.*

In an experimental trial, when the combination product was administered to seronegative dogs, which were later experimentally infected with the Leishmania parasite, the immune response exerted by the combination product in the dogs comprised a specific cytotoxic T-lymphocyte-mediated (CTL) response against the Leishmania antigens of the product and was able to promote parasite clearance in the bone marrow, defined as no parasites detectable by PCR in 90% of the vaccinated and infected dogs, 64 weeks after the infection (**Figure 6**). The parasite clearance correlated with the absence of clinical symptoms of Leishmaniasis in the vaccinated dogs 64 weeks after infection (**Figure 5**). In contrast, dogs immunized with only one of the two agents of the combination product, the chimeric protein PFR1-Hsp70, showed a negative PCR only in 60% of the of the vaccinated and infected dogs, 64 weeks after the infection (**Figure 6**) and, consistently, the dogs with a relatively high parasite load showed clinical symptoms of Leishmaniasis 64 weeks after infection (**Figure 5**) (**Table 3**).

In a different experimental trial, when the combination product was administered to seropositive and asymptomatic dogs, the immune response exerted by the product in the dogs was able to promote a significant (p<0.05) decrease of the mean parasite load detectable by PCR in the bone marrow, 35 weeks after the infection (**Figure 11**). Consistently, 15 months after infection of the dogs, and 10 months after treatment with the combination product, only 31% of the treated dogs showed clinical symptoms compatible with a Stage 2 of Leishmaniasis disease while 79% of the control untreated dogs reached this stage (**Figure 12**).

### Examples

### Example 1: Cloning of the first and second agent into an expression cassette

### 1.1. Cloning of the first agent into an expression cassette

Hsp70 coding sequence was amplified by PCR using *Trypanosoma cruzi* genomic DNA as template and specific primers. PFR1 coding sequence was amplified by PCR using *Leishmania infantum* genomic DNA as template and specific primers. The amplified Hsp70 and PFR1 fragments were cloned into individual pGEMT vectors. Clones containing pGEMT vectors with Hsp70 and PFR1 fragments were identified by restriction enzyme analysis using specific restriction enzymes. In addition, selected clones were sequenced to confirm identity of the sequence of interest. Hsp70 fragment cloned into pGEMT vector was subsequently digested with specific restriction endonucleases and the fragment was directly cloned into pQE32 vector digested with the same enzymes. The resulting construct was sequenced and named pQE32-HSP70. PFR1 fragment cloned into pGEMT vector was subsequently digested with BamHI and Asp718 restriction endonucleases and the fragment was directly cloned into pQE32-HSP70 vector digested with the same enzymes. The resulting construct was sequenced and named pQE32-PFR1-HSP70.

### 1.2. Cloning of the second agent into an expression cassette

Fragments of KMP11, PSA and TGP63 ORFs were amplified by PCR using genomic DNA of *Leishmania infantum* and specific primers, while fragment of 2 fII HSPTc was amplified by PCR using genomic DNA of *Triypanosoma cruzi* and specific primers. Q5 chimera sequence was constructed by amplification of KMP11, PSA, TGP63 and 2 fII HSPTc fragments by polymerase chain reaction (PCR) as follows. KMP11 fragment was amplified by PCR using genomic DNA of *Leishmania infantum* and specific primers containing BamH1 and SpH1 restriction sites. Amino PSA fragment was amplified by PCR using genomic DNA of *Leishmania infantum* and specific primers containing SpH1 and Kpn1 restriction sites. Carboxyl PSA fragment was amplified by PCR using genomic DNA of *Leishmania infantum* and specific primers containing Kpn1 restriction site. TGP63 fragment was amplified by PCR using genomic DNA of *Leishmania infantum* and specific primers containing KpnI and HindIII restriction sites. 2 fII HSP fragment was amplified by PCR using genomic DNA of *Triypanosoma cruzi* and specific primers containing HindIII restriction site. The amplified fragments were cloned into individual pGEMT vectors. Clones containing pGEMT vectors with KMP11, PSA, TGP63 and 2 fII HSPTc fragments were identified by restriction enzyme analysis using specific restriction enzymes. In addition, selected clones were sequenced to confirm identity of sequence of interest. KMP11, PSA, TGP63 and 2 fII HSPTc fragments cloned into pGEMT vectors were digested with specific restriction endonucleases and the fragments were cloned consecutively into pQE30 vector digested with the same enzymes in the order above mentioned. The resulting construct, named pQE30-Q5 (pQE30-KMP11-PSA-TGP63-2fII HSPTc) was sequenced to validate the sequence.

### Example 2: Expression of the first and second agent in Escherichia coli

### 2.1. Expression of the first agent in Escherichia coli

E. coli M15 transformed with pQE32-PFR1-HSP70 were inoculated in 7 mL of LB medium with ampicillin (100 µg/mL) and kanamycin (25 µg/mL). Cells were cultured overnight at 37°C and 220 rpm shaking. 5 mL of the overnight cultured cells were inoculated in 250 mL of LB medium with ampicillin (100 µg/mL) and kanamycin (25 µg/mL) and incubated at 37°C with 220 rpm shaking up to reach 0.2-0.3 O.D units at 600 nm. Induction of antigen expression was performed by adding IPTG (0.02 mM final concentration) and incubating for 3 hours at 37°C with 220 rpm shaking. Cells were collected by centrifugation at 9,820 g for 20 min and total proteins were solubilized in solubilization buffer 10 mM Na2HPO4, 60 mM NaCl, 1 phenyl methyl sulfonyl fluoride (PMSF), 0.05% SDS pH 8.0 by sonication.

### 2.2. Expression of the second agent in Escherichia coli

E. coli M15 transformed with pQE30-Q5 vector were inoculated in 85 mL of LB medium with ampicillin (100 µg/mL) and kanamycin (25 µg/mL). Cells were cultured overnight at 37°C and 220 rpm shaking. 12.5 mL of the overnight cultured cells were inoculated in 237.5 mL of LB medium with ampicillin (100 µg/mL) and kanamycin (25 µg/mL) and incubated at 37°C with 220 rpm shaking up to reach 0.2-0.4 OD units at 600 nm. Induction of Q5 expression was performed by adding 0.02 mM of isopropyl-beta-D-thiogalactopyranoside (IPTG) and incubating for 3 hours at 37°C with 220 rpm shaking. Cells were collected by centrifugation at 9,820 g for 20 min and total proteins were solubilized in solubilization buffer (50 mM Na2HPO4, 0.3 M NaCl, 1 mM PMSF, 0.05% SDS pH 8.0) by sonication.

### Example 3: Purification of the first and second agent using affinity chromatography

### 3.1. Purification of the first agent using affinity chromatography

PFR1 was subsequently purified to homogeneity under native conditions by Ni2+-NTA affinity chromatography to a 6× histidine tag placed at PFR1. After washing with 50 mM NaH2PO4, 0.3 M NaCl, 0.05% SDS, 10 mM 2-mercaptoethanol, 10% glycerol, 1 mM PMSF pH 8.0 and 50 mM NaH2PO4, 0.3 M NaCl, 0.05% SDS, 10 mM 2-mercaptoethanol, 1 mM PMSF pH 7.0, the protein was eluted in elution buffer (0.3 M NaCl, 50 mM NaH2PO4, 0.05%SDS, 1 mM PMSF, at pH 6.0). The final elution fractions resulting from the purification process were analysed by 10% SDS-PAGE and Coomassie Blue staining. The protein concentration was measured with a Micro BCA Protein Assay Kit (Thermo). Figure 1 shows the purified first and second agents.

### 3.2. Purification of the second agent using affinity chromatography

Recombinant Q5 was subsequently purified to homogeneity under native conditions by Ni2+-NTA affinity chromatography to a 6× histidine tag placed at Q5. After washing with 50 mM NaH2PO4, 0.3 M NaCl, 0.05% SDS, 10 mM 2-mercaptoethanol, 1 mM PMSF pH 8.0 and 50 mM NaH2PO4, 0.3 M NaCl, 0.05% SDS, 10 mM 2-mercaptoethanol, 1 mM PMSF pH 7.5, the protein was eluted in elution buffer (50 mM NaH2PO4, 0.3 M NaCl, 0.05%SDS, 1 mM PMSF, at pH 6.0). The final elution fractions resulting from the purification process were analysed by 10% SDS-PAGE and Coomassie Blue staining. The protein concentration was measured with a Micro BCA Protein Assay Kit (Thermo). Figure 1 shows the purified first and second agent. Both proteins were observed as major bands at the expected relative mass respect to the reference markers in their respective SDS-PAGE analysis.

### Example 4: Safety and efficacy trial of the combination product as a prophylactic vaccine against leishmaniasis in Beagle dogs

The study was carried out at the University of Murcia in southeast Spain, in a cohort of 32 *Leishmania-*free Beagle dogs, 16 of each gender, acquired from a commercial breeder (Isoquimen SL, Barcelona, Spain). They were randomly assigned into 6 experimental groups, four vaccinated groups (G1 to G4) and two non-vaccinated groups (G5 and G6). Vaccination treatments included the two different antigen described in the next section. In G1 (n=5) the dogs were immunized with 70 µg of the first agent (AG1) and Quil-A , i.e. an adjuvant. In G2 (n=5) the dogs were immunized with 70 µg of AG1 and 70 µg of the second agent (AG2) without adjuvant. In G3 (n=5) the dogs were immunized with 70 µg of AG1, 70 µg of AG2, and Quil-A in the primary immunizations and then without adjuvant in the subsequent booster immunizations. In G4 (n=5) the dogs were immunized with 70 µg of AG1, 70 µg of AG2, Quil-A, and IMP peptide in the primary immunizations and then without adjuvant in the subsequent booster immunizations. In G5 (n=5) the dogs were only immunized with Quil-A. In G6 (n=7) the dogs were not immunized. Details of the groups can be found in Figure 2.

Primary immunizations and booster immunizations were administered using the immunization schedule outlined in Figure 3. The vaccination protocol included three initial doses administered at three week intervals in June and July 2014, and a booster inoculation in March 2015, one month later all dogs were infected with *L. infantum* in April 2015 as described below. Dogs were physically examined in the three days that followed each vaccination dose to detect adverse reactions and 1 time thereafter until the end of the experiment in July 2016 to assess their clinical status. Blood samples for clinico-pathological analysis were taken after each vaccination and each time they were examined after infection, in order to classify dogs as healthy or with CanL, as described below. Serum was also used to analyze the dog's *L. infantum* antibody status and bone marrow samples were taken to investigate the dog's infection-status by real-time PCR (rtPCR) as later detailed. Figures 3, 5 and 6 show the chronology of all interventions.

Since arriving in Murcia, dogs had unlimited access to a chicken-base diet and were housed in open-air, partially covered pens at 5m²/dog, in a *L. infantum-endemic,* periurban area. These were the only available premises to do the trial, and they are situated in an area where the estimated prevalence of subclinical *L. infantum* infection in dogs was 67% (Chitimia et al., 2011), similar to other endemic areas in Spain (Solano-Gallego et al., 2009). To limit the risk of external *L. infantum* infection, dogs were treated preventively every 3 weeks with a sand fly licensed topical insect repellent (Advantix^{®} spot-on pipettes, Bayer^{®}), containing permethrin (500 mg/ml) and imidacloprid (100 mg/ml). The humoral response of dogs to the salivary antigens of *L. infantum* vector, *Phlebotomus perniciosus* and the other main sand fly in the area, *P. papatasi,* was monitored periodically as previously reported (Risueño et al., 2018). Other treatments administered to prevent parasitic and infectious diseases included a broad-spectrum anthelmintic (Prazitel, Ecuphar^{®}) every three months and annual vaccination courses against rabies, distemper, adenovirus 2, parvovirus, *Leptospira interrrogans* (Eurican MHPPi2, Merial^{®}) and *Bordetella bronchiseptica* and Parainfluenza virus (Eurican Bb/PI2, Merial^{®}).

### 2.2. Vaccine antigens

The vaccine candidates evaluated in this study included the BNT005 protein with the PFR1-HSP70 antigen (antigen 1) and the Q5 chimera containing the KPM11 fragment of Parasite Surface Antigen 2 (PSA2), Tgp63 and HSP70 (antigen 2).

The recombinant PFR1 protein was overexpressed by adding 0.02 mM of IPTGto the E. coli M15 strain transformed with pQE32-PFR1-HSP70 and grown for 3 h at 37 °C. Total proteins were solubilized in solubilization buffer (0.3 M NaCl, 50 mM Na2HPO4, 0.05%SDS, 1 mM PMSF, pH 8.0) and by sonication. Recombinant PFR1 was subsequently purified to homogeneity under native conditions by Ni2+-NTA affinity chromatography and eluted in elution buffer (0.3 M NaCl, 50 mM NaH2PO4, 0.05%SDS, 1 mM PMSF, at pH 6.0). The recombinant Q5 protein was overexpressed by adding 0.02 mM IPTG to the E. coli M15 strain transformed with pQE30-Q5 and grown for 3 h at 37 °C. Total proteins were solubilized in solubilization buffer (0.3 M NaCl, 50 mM Na2HPO4, 0.05%SDS, 1 mM PMSF, pH 8.0) and by sonication. Recombinant Q5 was subsequently purified to homogeneity under native conditions by Ni2+-NTA affinity chromatography and eluted in elution buffer (0.3 M NaCl, 50 mM NaH2PO4, 0.05%SDS, 1 mM PMSF, at pH 6.0). The final elution fractions resulting from the purification process were analysed by 10% SDS-PAGE and Coomassie Blue staining. The protein concentration was measured with a Micro BCA Protein Assay Kit (Thermo). Figure 1 shows the purified first and second agent.

### 2.3. Assessment of dog's infection status prior to infection and experimental infection with L. infantum

Prior to infection, dogs were confirmed negative for *L. infantum* DNA in blood and bone marrow samples analysed with the real time Polymerase Chain reaction (rtPCR) test described below, at the time of joining the experiment in 2014 and just before they were experimentally infected in April 2015. In addition, dogs had tested negative to *L. infantum* serum antibodies when analysed by the breeder before delivering the dogs using an indirect immunofluorescence test, and when they received the first *L*. *infantum* vaccination dose, when tested with the Enzyme-Linked ImmunoSorbent Assay (ELISA) below.

Dogs were experimentally infected by intravenous injection of 1×10⁶ stationary-phase promastigotes (infective promastigotes) of *L. infantum* (MCAN/BR/00/BA262). These parasites were isolated from spleen of an experimentally infected hamster in Schneider's Drosophila medium (Biowest) supplemented with 10% of inactivated fetal bovine serum (iFBS) and 50 µg/mL gentamicin at 26°C. Promastigotes transformed from spleen-derived amastigotes were cultured in modified RPMI-1640 medium supplemented with 20% of iFBS, and 50 µg/mL gentamicin at 26°C. The experimental infection was confirmed by serological tests against *Leishmania* antigens, and by real-time *Leishmania* kinetoplast-specific PCR.

### Example 5: Clinical and serological evolution of Beagle dogs after experimental infection with Leishmania infantum promastigotes

The dogs were vaccinated with 3 doses in 3 weeks intervals, receiving the last dose 9 months before challenge (according to the calendar and groups description in Figure 3). One month before the experimental infection, dogs of the vaccinated groups were boosted. Clinical and serological evaluations of dogs were made every month after infection. Clinical and serological evolution of Beagle dogs is summarized in Figure 5. The legend shows several clinical symptoms (1 to 6), hematological & biochemical symptoms (7 to 10) or acute protein indicators related with the leishmaniasis disease. Grey scale color code indicates the health status of the dogs, from light to dark grey according to the severity. Rows marked with (*) correspond to dogs that did not show any symptom of disease along the study period.

At the end of the experiment, 5/7 (71%) of dogs in the control group G6 had an advanced clinical stage of the disease (Figure 4). 2/5 (40%) of the dogs in the group vaccinated just with AG1 (G1) showed clinical symptoms of the disease. On the other hand, at the end of the experiment, any of the dogs (0%) in the group vaccinated with the combination product of the present invention (G3 and G4) showed clinical symptoms of infection, only hematological or biochemical parameters were detected.

The results show that vaccination with the combination product of the present invention reduced the clinical symptoms of leishmaniasis caused by experimental infection up to 0%, improving the performance of AG1 (40%) and control group (71%) (**Table 3**).

### Example 6: Determining the parasite load using qPCR

Parasite load was determine using a Taqman-based quantitative PCR assay targeting an approximately 140 bp kinetoplast sequence using DNA from bone marrow aspirates samples as template. Every sample was analyzed in duplicate using 300 ng of high quality DNA template (nanodrop A260/A280 ratio ≥ 1.7) per reaction. The amount of parasite DNA present in the samples was estimated using the threshold amplification cycle (Ct) that corresponds to the cycle at which near-logarithmic amplification occurred and is inversely related to the amount of parasite DNA present. Ct>38 were deemed negative.

As can be seen in Figure 6, 3/5 (60%) of the dogs vaccinated with Antigen 1 (G1) did not show detectable levels of *Leishmania* DNA in the bone marrow 15 months after the challenge. In contrast, 9/10 (90%) of the dogs vaccinated with the combination product of the present invention (G3 and G4) did not show detectable levels of *Leishmania* DNA in the bone marrow, improving the performance of G1. On the other hand, only 1/7 (14%) of the dogs in control group G6 did not show detectable levels *of Leishmania* DNA in the bone marrow.

The results showed that the combination product was able to promote parasite clearance in the bone marrow, defined as no parasites detectable by PCR in 90% of the vaccinated and infected dogs, 64 weeks after the infection, improving the performance of AG1 (60%) and control group (14%) (**Table 3**).

### Example 7: ELISA to quantify the amount of anti-AG1 and anti-AG2 antibodies in serum

Total IgG anti-AG1 and Total IgG anti-AG2 were measured in serum samples. OD values were measured in a direct ELISA coated by Leishmania infantum PFR1 (AG1) or Q5 (AG2) proteins expressed and purified from E. coli.

Figures 7 and 8 show that all animals in vaccinated groups responded to vaccination by creating specific humoral immunity against the antigens included in the vaccine formulation assigned to each group. No effect was observed in the animals of the only adjuvant group G5. Figure 7 shows that dogs which were immunized with Quil-A contained little or no ant-PFR1 antibodies in the serum. However, immunization with AG1 either alone or in combination with AG2 led to an increase in anti-PFR1 antibodies in the serum. AG1 combined with AG2 without adjuvant produced the lowest antibody titer whereas AG1 combined with Quil-A produced the consistently highest titer. AG1 and AG2 in combination with adjuvant produced comparable titers to that of the AG1 combined with Quil-A.

Figure 8 shows that dogs which were immunized with Quil-A or AG1 with Quil-A contained little or no anti-AG2 antibodies in the serum. Out of all the dogs, those in G3 and G4 contained the largest titer of anti-AG2 antibodies.

### Example 8: Proliferation of peripheral blood mononuclear cells after stimulation with AG1 or AG2

PBMCs isolated from dogs were cultured and stimulated with the antigens AG1 and AG2, expressed and purified from E. coli, and the known lymphocyte mitogen concanavalin A (ConA), which was used as positive control. Cell proliferation was measured by adding a radioactive marker of growth 120 hours after stimulation with antigens. The incorporated radioactivity was quantitated 20 hours later. The stimulation index was calculated as follows: s.i. = (radioactivity measured in stimulated cells - radioactivity measure in unstimulated cells) / radioactivity measured in unstimulated cells.

The cells were obtained from the dogs (D1-D23; 3 dogs per group G1 to G5) either before immunization (S0), 3 weeks after each immunization (S1, S2 and S3 respectively) or at 11 and 29 weeks after the last immunization (S4 and S5 respectively).

All vaccinated dogs showed specific proliferation when stimulated with the antigens used in the vaccine formulations. Figure 9 shows that the capacity of PFR1 of stimulating PBMCs, indicative of the presence of anti-PFR1 T cell memory is well established after the second dose of the primo-vaccination and is lasting at least until 29 weeks post 3rd immunization. Similar results were obtained after stimulation of the PBMCs with the AG2.

Immunization of the dogs with AG1 lead to the PBMCs being sensitive to stimulation by PFR1. Immunization of the dogs with AG1 and AG2 lead to the PBMCs being sensitive to stimulation by PFR1 and AG2. Moreover, immunization of AG1 and AG2 without adjuvant was enough to make the cells sensitive to stimulation by both AG1 and AG2.

### Example 9: Expression of cytokines by PBMCs after stimulation with AG1 or AG2

Expression of cytokines TNF-α, IL-6, IL10, IFN-Y, MCP-1 and GM-CSF by PBMCs were determined in cell culture supernatants by using a Canine Cytokine Milliplex kit after stimulation with the antigens PFR1 (upper panel) or AG2 (lower panel). The cells were obtained from the dogs before immunization (S0), 3 weeks after each immunization (S1, S2 and S3 respectively), 11 and 29 weeks after the last immunization (S4 and S5 respectively). The cell supernatant was harvested at either 24 h for determination of concentrations of cytokines TNF-α, IFN-γ, IL-6 and IL-10, or 48 h for determination of concentrations of cytokines MCP-1 and GM-CSF. An "X" in the panel means a sample value could not be determined because of technical problems.

Figure 10 shows a summary of the quantitative measurements of the panel of 6 cytokines/chemokines assayed in the supernatants of PBMC obtained from the dogs. PBMCs were stimulated by either AG1 or AG2. Significant release of all the cytokines assayed were detected in samples from all dogs, including dogs of the only adjuvant group (G5). However, the levels of cytokines detected in samples from group G5 were lower than those obtained in the majority of the vaccinated dogs.

High levels of MCP-1, which has been linked to clearance of Leishmania parasites in humans, were detected in 11 out of 12 vaccinated dogs (92%). The pattern of accumulation of this cytokine in the supernatant of cells stimulated by either AG1 or AG2 was very similar. MCP-1 has shown synergy with IFN-γ in promoting the type of Th1 response linked to resolution of Leishmania infection. High levels of IFN-γ were detected in 4 out of the 6 dogs (67%) immunized with both antigens plus adjuvant (groups G3, G4), in which IFN-γ was released after stimulation by either PFR1 or AG2 (Figure 10). Only 1 dog of G1 showed significant release of IFN γ. Similarly, the cells from only 1 of the dogs of G2 showed relatively high levels of IFN-γ. The pro-inflammatory cytokine TNF-α has been recognized as one of the key components of the Th1-type cellular response against L. infantum. PBMCs from 11 out of the 12 (98%) Vaccinated dogs (groups G1-G4) showed high release of TNF-α when stimulated with PFR1. Similarly, mononuclear cells from all dogs vaccinated with formulations including AG2 released high levels of TNF-α. Importantly, detection of TNF-α correlated with relatively low levels of the antiinflammatory cytokine IL-10, which is consistent with TNF-α participating in activation of a Th1-type response. IL-6 was also released by the PBMCs obtained from all vaccinated dogs, either when stimulated AG1 or AG2. IL-6 levels released by mononuclear cells from 56% of the dogs vaccinated with both antigens (G2, G3, G4) were significantly higher than those corresponding to the unspecific effect observed in mononuclear cells obtained from dogs of the only adjuvant group (Figure 10).

Taken together, these results support the combination product vaccine candidate as immunogen able to raise an immune response against different antigenic proteins of Leishmania infantum, characterized by a long-lasting Th1-type humoral and cellular response, involving cytotoxic T lymphocytes.

## Claims

1. A combination product which comprises:
a. a first agent capable of leading to the antigenic presentation of a polypeptide comprising or consisting of the amino acid sequences:
i. SEQ ID NO: 1 or a functional variant thereof which has at least 75 %, 80 %, 85 %, 90 %, 95 %, 96 %, 97 %, 98 %, or 99 % sequence identity with SEQ ID NO: 1, and
ii. SEQ ID NO: 3 or a functional variant thereof which has at least 75 %, 80 %, 85 %, 90 %, 95 %, 96 %, 97 %, 98 %, or 99 % sequence identity with SEQ ID NO: 3,
wherein both sequences (SEQ ID NO 1 and 3) are directly linked or optionally separated by a linker such as the linker set forth in SEQ ID NO: 2; and
b. a second agent capable of leading to the antigenic presentation of a polypeptide comprising or consisting of the amino acid sequences:
i. SEQ ID NO: 4 or a functional variant thereof which has 75 %, 80 %, 85 %, 90 %, 95 %, 96 %, 97 %, 98 %, or 99 % sequence identity with SEQ ID NO: 4 directly linked, optionally through a linker such as the linker set forth in SEQ ID NO. 5, with
ii. SEQ ID NO: 6 or a functional variant thereof which has 75 %, 80 %, 85 %, 90 %, 95 %, 96 %, 97 %, 98 %, or 99 % sequence identity with SEQ ID NO: 6, which in turn is directly linked, optionally through a linker such as the linker set forth in SEQ ID NO: 7, with
iii. SEQ ID NO: 8 or a functional variant thereof which has 75 %, 80 %, 85 %, 90 %, 95 %, 96 %, 97 %, 98 %, or 99 % sequence identity with SEQ ID NO: 8, which in turn is directly linked, optionally through a linker such as the linker set forth in SEQ ID NO: 9, with
iv. SEQ ID NO: 10 or a functional variant thereof which has 75 %, 80 %, 85 %, 90 %, 95 %, 96 %, 97 %, 98 %, or 99 % sequence identity with SEQ ID NO: 10, which in turn is directly linked, optionally through a linker such as the linker set forth in SEQ ID NO: 11, with
v. SEQ ID NO: 12 or a functional variant thereof which has 75 %, 80 %, 85 %, 90 %, 95 %, 96 %, 97 %, 98 %, or 99 % sequence identity with SEQ ID NO: 12.

2. The combination product of claim 1, wherein the combination product comprises:
a. a first agent capable of leading to the antigenic presentation of a polypeptide comprising or consisting of the amino acid sequence set forth in SEQ ID NO: 13 or a polypeptide which has at least 75 %, 80 %, 85 %, 90 %, 95 %, 96 %, 97 %, 98 %, or 99 % sequence identity with SEQ ID NO: 13, and
b. a second agent capable of leading to the antigenic presentation of a polypeptide comprising or consisting of the amino acid sequences set forth in SEQ ID NO: 14 or a polypeptide which has 75 %, 80 %, 85 %, 90 %, 95 %, 96 %, 97 %, 98 %, or 99 % sequence identity with SEQ ID NO: 14.

3. The combination product of claim 2, wherein the combination product comprises:
a. a first agent capable of leading to the antigenic presentation of a polypeptide comprising or consisting of the amino acid sequence set forth in SEQ ID NO: 13, and
b. a second agent capable of leading to the antigenic presentation of a polypeptide comprising or consisting of the amino acid sequences set forth in SEQ ID NO: 14.

4. The combination product according to any one of claims 1-3, wherein the combination product further comprises an adjuvant, preferably Quil-A^{®}.

5. The combination product according to any one of claims 1-4, wherein the combination product is a composition; or the first agent and the second agent are physically separated.

6. The combination product according to any one of claims 1-4, wherein the combination product comprises:
(a) the first agent and instructions on how to administer the first agent with the second agent; or
(b) the second agent and instructions on how to administer the second agent with the first agent.

7. The combination product according to any one of claims 1-6, wherein the combination product is prepared for oral, sublingual, buccal, intravenous, intramuscular, subcutaneous, rectal, transdermal, topical and/or inhalation-mediated administration.

8. The combination product according to any one of claims 1-7, wherein the combination product is a vaccine capable of inducing an immune response.

9. The combination product for use according to claim 8, wherein the response induced is a humoral immune response, CD8+ T cell-mediated immune response and/or a CD4+ T cell-mediated immune response against the *Leishmania* antigens *PFR1, KMP11, PSA* and *gp63.*

10. The combination product according to any one of the preceding claims for use as a medicament.

11. The combination product according to any one of claims 1-9, for use in the treatment and/or prevention of infections and/or diseases caused by parasites of the genus *Leishmania.*

12. The combination product for use according to claim 11, wherein the combination product is used to treat humans and/or animals.

13. The combination product for use according to any one of claims 11-12, wherein the combination product is used to treat dogs.

14. The combination product for use according to any one of claims 11-13, wherein the parasite of the genus *Leishmania* is of the species *Leishmania infantum.*

15. A kit for the treatment and/or prevention of infections and/or diseases caused by parasites of the genus *Leishmania* comprising the combination product according to any one of claims 1-9.
